# FASCICULE DE BREVET EUROPEEN

(11) **EP 2 496 141 B1**
(45) Date de publication et mention de la délivrance du brevet: **24.07.2013**
(21) Numéro de dépôt: 10785096.8
(22) Date de dépôt: 29.10.2010
(51) Int. Cl.: A61B 5/155

(54) **DISPOSITIF DE PRELEVEMENT DE LIQUIDE CORPOREL ET PROCEDE DE MISE EN OEUVRE.**
VORRICHTUNG ZUR ENTNAHME EINER KÖRPERFLÜSSIGKEITSPROBE UND VERFAHREN ZU IHRER ANWENDUNG
DEVICE FOR TAKING A SAMPLE OF A BODY FLUID AND METHOD FOR IMPLEMENTING SAME

(30) Priorité: 05.11.2009 FR 0905330
(43) Date de publication de la demande: 12.09.2012
(73) Titulaire: Centre National de la Recherche Scientifique, 75016 Paris (FR); Centre Hospitalier Universitaire de Besançon, 25030 Besançon Cedex (FR); Université de Franche-Comté, 25000 Besançon (FR); Etablissement Français du Sang, 93218 La Plaine Saint Denis Cedex (FR)
(72) Inventeur: PAZART, Lionel, Henri, F-25000 Besancon (FR); WACOGNE, Bruno, François, Marcel, F-70190 Traitiefontaine (FR); PIERALLI, Christian, Gérard, Daniel, F-25000 Besancon (FR); BOIREAU, Wilfrid, F-25680 Mondon (FR); MOREL, Pascal, Charles, Serge, F-25000 Besancon (FR)
(74) Mandataire: Rataboul, Xavier
(86) Numéro de dépôt international: PCT/FR2010/000712
(87) Numéro de publication internationale: WO 2011/055029

(56) Documents cités:
- WO-A2-2006/088771
- US-A- 4 014 328
- US-A- 4 865 583
- US-A- 4 981 140
- US-A- 5 372 143

## Description

L'invention se rapporte à un dispositif de prélèvement de liquide corporel, en particulier du sang; et à un procédé de mise en oeuvre.

Lorsqu'un patient (humain ou animal) est perfusé, il peut être nécessaire de lui faire un prélèvement, tel qu'une prise de sang, pour analyse. Dans ce cas, une personne autorisée (médecin, infirmière ou vétérinaire) réalise :
- soit un prélèvement indépendant de la perfusion. Le patient subit donc une première piqûre pour la pose de la perfusion, puis une deuxième piqûre pour le prélèvement.
- soit un prélèvement au moment de la mise en place du cathéter (ou de son changement), avec une connexion directe sur le cathéter, avant de mettre en place la ligne de perfusion,
- soit un prélèvement, à l'aide d'une seringue, à partir de la rampe de robinet du prolongateur (habituellement de quelques dizaines de cm de long) intercalé entre la tubulure de perfusion et le cathéter. Dans ce cas, il est nécessaire de faire une première manipulation de purge (aspiration entre 1 et 7,5 ml selon les pratiques et le type d'analyse de sang à réaliser) puis de réaliser le prélèvement sanguin proprement dit. La réinjection de l'aspiration de purge est en général réalisée assez rapidement dans le prolongateur, afin d'éviter un risque d'hémolyse.

L'invention s'applique principalement mais non exclusivement aux perfusions sur voies veineuses centrales ou périphériques.

De manière connue, une perfusion sur une veine périphérique est mise en place de la manière suivante.

Après avoir repéré une veine périphérique, on pique la peau du patient à l'aide d'une aiguille enrobée d'un cathéter (un tel dispositif est connu sous la marque Cathlon®). Une fois l'aiguille positionnée dans une veine, on pousse l'extrémité distale du cathéter dans la veine, on retire l'aiguille, et on fixe l'extrémité proximale du cathéter sur la peau du patient par un pansement stérile. Différents diamètres de cathéter existent, et sont généralement exprimés en Gauge(G). Le diamètre du cathéter utilisé dépend de l'état du patient, de l'opération à réaliser (prélèvement ou injection) et de l'âge du patient. Par exemple, on choisira un cathéter de 22 à 24 Gauge pour les nouveau-nés, de 22 Gauge pour les enfants de 1 mois à 3 ans, de 20 gauge pour les enfants plus grands. Pour les adultes, on pourra choisir des cathéters de 18 Gauge (soit un diamètre externe de 1,1 mm à 1,3 mm) jusqu'à 14 Gauge (soit un diamètre externe de 1,8 à 2,2 mm).

Une table de correspondance entre l'unité « Gauge » et le système métrique est donné ci-après :

| Gauge (diamètre interne de l'aiguille) | Diamètre externe (mm) |
|---|---|
| 24 G | 0,6 à 0,7 |
| 22 G | 0,7 à 0,9 |
| 20 G | 0,9 à 1,1 |
| 18 G | 1,1 à 1,3 |
| 16 G | 1,5 à 1,8 |
| 14 G | 1,8 à 2,2 |

Le cathéter porte, à son extrémité proximale, un dispositif de connexion, par exemple de type luer-lock, à l'extrémité distale d'une tubulure de perfusion. Ce dispositif de connexion peut être tel que lorsque le cathéter n'est pas connecté, son extrémité proximale est fermée et le sang ne peut pas sortir.

En général, après la connexion au cathéter, on réalise une boucle de sécurité avec la tubulure que l'on fixe par un adhésif sur la peau du patient. Cette boucle de sécurité évite l'arrachage immédiat du cathéter en cas de traction sur la tubulure de perfusion.

L'extrémité proximale de la tubulure est en communication fluidique avec un vase d'expansion connecté à une poche rigide ou souple de produit de perfusion fixée à une perche. Cette dernière doit être suffisamment haute par rapport au cathéter du patient pour que le produit de perfusion s'écoule par simple gravité vers le cathéter puis le système vasculaire du patient.

La tubulure comprend, de préférence, une molette de réglage du débit de perfusion, ou autre système de régulation.

Il est connu de vérifier que le cathéter est bien positionné dans le système vasculaire du patient en détachant la poche de perfusion de sa perche et en la baissant à une hauteur inférieure à celle du cathéter du patient. Dans ces conditions, la pression sanguine est supérieure à la pression du produit de perfusion. Si le cathéter est bien positionné, on peut observer un reflux de sang dans la tubulure de perfusion.

Pour éviter de faire une deuxième piqûre à un patient perfusé, le document WO2006/088771 propose de munir une perfusion classique d'une pompe réversible associée à un moyen de commande. Ce dernier est conçu pour interrompre, par intermittence, le fonctionnement de la pompe à perfusion dans le sens avant (c'est-à-dire depuis la poche de produit de perfusion vers le patient), afin de faire fonctionner la pompe dans le sens arrière (c'est-à-dire depuis le patient vers un circuit de prélèvement). De cette manière, il est possible de prélever du sang du patient par le cathéter de perfusion.

Cependant, ce dispositif nécessite un réglage minutieux de la pompe et un circuit fluidique complexe comprenant de multiples vannes.

En outre, ce système peut être bruyant (en raison du fonctionnement de la pompe) et nécessite une source d'énergie pour le prélèvement de sang et l'injection du produit de perfusion.

Un premier objectif de l'invention est de proposer un dispositif simple, efficace et économe en énergie, permettant de réaliser de manière aisée un prélèvement de liquide corporel en évitant un nouveau geste intrusif dans le corps du patient, et en limitant la nécessité d'une purge préalable au prélèvement.

D'autre part, lorsqu'un patient (humain ou animal) est perfusé, par exemple avec un produit thérapeutique (solution saline, solution antibiotique, etc.), il peut être nécessaire de lui faire une deuxième perfusion d'un autre produit, tel que du sang, après que le médecin ait diagnostiqué une situation médicale donnée (maladie, accident, hémorragie, etc.). Dans l'exemple décrit, il s'agit d'un besoin de sang.

Dans ce cas, une personne autorisée (médecin, infirmière ou vétérinaire) doit installer une deuxième ligne de perfusion, spécifique pour la transfusion de sang.

Classiquement pour une voie sur une veine périphérique, la deuxième perfusion est installée sur le bras controlatéral. Le patient subit donc une première piqûre pour la pose de la première perfusion, puis une deuxième piqûre pour la pose de la deuxième perfusion.

Avant de faire circuler le produit de la deuxième perfusion, il est essentiel d'effectuer un contrôle ultime de compatibilité entre le traitement prescrit par le médecin et le traitement que l'on s'apprête à donner au patient via la deuxième perfusion.

Par exemple, pour la transfusion sanguine, lorsqu'un médecin a prescrit une transfusion pour un patient de groupe sanguin donné, l'infirmière doit s'assurer que les poches de sang dont elle dispose sont compatibles avec le groupe sanguin du patient car il peut exister des erreurs d'attribution des poches de sang ou d'identification du patient ou des poches de sang.

Classiquement, l'infirmière utilise un papier cartonné imprégné de réactif anti-A et anti-B sur lequel elle dépose du sang du patient (obtenu par une piqûre au bout du doigt ou dans une veine) et du sang issu d'un échantillon de la poche de transfusion. A cette fin, les poches de transfusion présentent un réservoir principal pour la transfusion, et des réservoirs secondaires d'échantillonnage, séparables du réservoir principal.

L'infirmière apprécie alors la présence d'agglutinats sur le papier et compare les réactions obtenues avec le sang du patient et avec le sang de l'échantillon. Elle doit alors appliquer les règles de compatibilité qu'elle a apprise pour interpréter les résultats du test. Cette interprétation peut être délicate, notamment en présence d'antigènes faibles ou dans certaines pathologies.

Ainsi, on s'est aperçu qu'il existe encore de nombreuses erreurs dues à la fatigue, à une situation d'urgence ou à une inattention, lors de l'interprétation de ces tests, mais également lors de l'installation de la poche de transfusion et, plus généralement, de la deuxième perfusion.

En particulier, on s'est aperçu qu'encore trop souvent le produit de la deuxième perfusion n'est pas celui qui avait été prescrit par le médecin.

Ces erreurs peuvent simplement ralentir la guérison du patient, par exemple lorsque le dosage du produit perfusé est inférieur à celui qui est prescrit par le médecin pour guérir le patient. Elles peuvent également entraîner la mort du patient, par exemple lorsque du sang transfusé est incompatible avec le groupe sanguin (A, B, AB ou O) du patient, ou lorsque le patient est allergique à l'antibiotique perfusé alors qu'il n'était pas allergique à l'antibiotique prescrit initialement.

Il peut également survenir des erreurs de manipulation dangereuses pour le personnel soignant, s'il y a une exposition directe au sang du patient.

Pour palier ce problème, les solutions actuelles visent à s'assurer de la concordance des informations inscrites sur la poche de transfusion et des informations inscrites sur un support porté par le patient. Ces technologies consistent, principalement, en des systèmes de contrôle à code-barres ou puce RFID. Cependant, il existe encore des erreurs de manipulation notamment en raison d'erreurs d'étiquetage des poches de perfusion ou d'identification des patients.

Un autre objectif de la présente invention est donc de proposer un système de perfusion permettant de réaliser de manière simple, efficace et économe en énergie, un contrôle ultime de compatibilité d'un traitement, préalablement choisi par un médecin, avec le patient et/ou la situation médicale préalablement diagnostiquée par un médecin.

Pour cela, l'invention propose de réaliser un système de perfusion comprenant un contrôle ultime sur la même ligne dé perfusion connectée au patient.

A cette fin, l'invention a pour objet un dispositif de prélèvement d'un liquide corporel, tel que du sang, destiné à être incorporé dans un circuit fluidique d'une perfusion d'un patient muni d'un cathéter de perfusion, et comprenant une structure tubulaire de connexion au circuit fluidique de la perfusion munie :
- d'une zone d'intubation, en utilisation, d'un canal de prélèvement comprenant une extrémité distale ; et
- d'un moyen de maintien, en utilisation, dans la structure tubulaire, de la partie du canal de prélèvement comprenant une extrémité distale, de sorte que ladite extrémité distale soit dirigée vers le cathéter de perfusion, dans le sens d'écoulement du produit de perfusion, depuis un réservoir de produit de perfusion vers le cathéter de perfusion.

Selon d'autres modes de réalisation :
● la partie comprenant l'extrémité distale du canal de prélèvement peut être fixée dans la structure tubulaire, et une partie comprenant une extrémité proximale du canal de prélèvement débouche hors la structure tubulaire au niveau de la zone d'intubation ;
● la zone d'intubation peut comprendre un moyen d'insertion adapté pour permettre, en utilisation, l'insertion, dans la structure tubulaire, de la partie du canal de prélèvement comprenant l'extrémité distale ;
● la zone d'intubation peut être agencée pour que, en utilisation, elle soit disposée à une distance verticale maximale déterminée de l'extrémité proximale du cathéter ;
● la distance verticale maximale déterminée peut être comprise entre 0 cm et 50 cm ;
● le moyen d'insertion peut être choisi parmi :
   - une membrane en un matériau étanche conservant son étanchéité après avoir été percé ; et
   - un connecteur étanche ;
● le matériau étanche conservant son étanchéité après avoir été percé peut être choisi parmi un polymère de silicone, tel que le polydiméthylsiloxane (PDMS), du polyméthylmétacrylate (PMMA), du Polychlorure de vinyle (PVC), et du Tygon® ; et/ou
● le dispositif de prélèvement peut comprendre, en outre :
   - un moyen de connexion à une extrémité proximale d'un cathéter de perfusion, et
   - un moyen de connexion à une extrémité distale d'une tubulure de perfusion.

L'invention se rapporte également à un cathéter de perfusion, comprenant :
- une extrémité distale destinée à être insérée dans un patient ;
- une extrémité proximale destinée à être connectée avec une extrémité distale d'une tubulure de perfusion
et comprenant, entre l'extrémité distale et l'extrémité proximale, un dispositif de prélèvement de liquide corporel précédent, agencé pour que, en utilisation, la zone d'intubation soit disposée à une distance verticale maximale déterminée de l'extrémité proximale du cathéter.

L'invention se rapporte également à une tubulure de perfusion, comprenant :
- une extrémité proximale destinée à être connectée à un réservoir d'un premier produit de perfusion ;
- une extrémité distale destinée à être connectée avec une extrémité proximale d'un cathéter de perfusion insérée dans un patient ;
et comprenant, entre l'extrémité proximale et l'extrémité distale, un dispositif de prélèvement de liquide corporel précédent, agencé pour que, en utilisation, la zone d'intubation soit disposée à une distance verticale maximale déterminée de l'extrémité proximale du cathéter.

L'invention se rapporte également à un kit de prélèvement d'un liquide corporel comprenant:
- un canal de prélèvement comprenant une extrémité proximale destinée à être reliée à un réservoir de prélèvement, et une extrémité distale, et
- un dispositif de prélèvement précédent.

Selon d'autres modes de réalisation :
● le kit peut présenter un rapport entre le diamètre intérieur du canal de prélèvement et le diamètre extérieur de- la- structure- tubulaire du dispositif de prélèvement inférieur à 1, typiquement compris entre 1/20 et 1/3 ;
● le canal de prélèvement peut présenter une longueur entre ses deux extrémités comprise entre 10 cm et 100 cm, de préférence entre 20 cm et 50 cm ;
● le kit peut comprendre, en outre, un moyen d'analyse du liquide corporel prélevé ;
● le moyen d'analyse peut être également apte à analyser un échantillon de produit déterminé et à comparer le liquide corporel prélevé et l'échantillon de produit déterminé ;
● le moyen d'analyse peut comprendre une chambre de réaction et un moyen de détection ;
● le kit peut comprendre, en outre, un moyen de commande d'écoulement d'une deuxième perfusion, le moyen d'analyse étant apte à transmettre au moyen de commande d'écoulement une information de comparaison du liquide corporel prélevé et de l'échantillon de produit déterminé ;
● le kit peut comprendre un moyen d'affichage de l'information de comparaison du liquide corporel prélevé et de l'échantillon de produit déterminé ;
● le moyen d'analyse peut être apte à contrôler le moyen de commande d'écoulement pour qu'il bloque l'écoulement du produit de la deuxième perfusion si le liquide corporel et le produit de la deuxième perfusion ne sont pas compatibles, et pour qu'il autorise l'écoulement du produit de la deuxième perfusion si le liquide corporel et le produit de la deuxième perfusion sont compatibles ;
● le moyen d'analyse peut être apte à contrôler le moyen de commande d'écoulement pour qu'il génère automatiquement l'écoulement du produit de la deuxième perfusion si le liquide corporel et le produit de la deuxième perfusion sont compatibles, et pour qu'il ne génère pas l'écoulement du produit de la deuxième perfusion si le liquide corporel et le produit de la deuxième perfusion ne sont pas compatibles ;
● le kit peut comprendre, en outre, un réservoir de prélèvement, comprenant, éventuellement, un moyen d'anti-coagulation du sang ;
● le réservoir de prélèvement peut être un réservoir à deux compartiments séparés par un système anti-retour, tel qu'une valve, un clapet, une bille, un flotteur etc. ;
● le réservoir de prélèvement peut être un réservoir déformable et actionnable mécaniquement, par exemple manuellement, tel qu'une poire d'évacuation-aspiration,
● le réservoir de prélèvement peut être mis préalablement en dépression sous vide et maintenu dans cet état par un clamp ;
● le kit peut comprendre un cathéter de perfusion précédent, muni du dispositif de prélèvement ; et/ou
● le kit peut comprendre une tubulure de perfusion précédente, munie du dispositif de prélèvement.

L'invention se rapporte également à un procédé de mise en oeuvre d'un kit de prélèvement précédent, comprenant les étapes suivantes :
- incorporer le dispositif de prélèvement dans un circuit fluidique d'une perfusion préalablement installée sur un patient muni d'un cathéter de perfusion, dans une partie externe du circuit fluidique par rapport au corps du patient ;
- maintenir, à l'intérieur de la structure tubulaire, la partie du canal de prélèvement comprenant l'extrémité distale, de sorte que ladite extrémité distale soit dirigée vers le cathéter de perfusion, dans le sens d'écoulement du produit de perfusion, depuis un réservoir de produit de perfusion vers le cathéter de perfusion ;
- amener le réservoir de prélèvement à une hauteur inférieure à celle du cathéter du patient, de telle sorte que le liquide corporel s'écoule dans le canal de prélèvement, à contre-courant par rapport au produit de perfusion puis vers le réservoir de prélèvement ; et
- maintenir le réservoir de prélèvement à une hauteur inférieure à celle du cathéter du patient pendant une durée suffisante pour obtenir, dans le réservoir de prélèvement, un volume suffisant pour constituer un prélèvement de liquide corporel.

Ce procédé permet de faire un prélèvement de liquide corporel sans avoir à réaliser une piqûre supplémentaire à un patient déjà perfusé.

Le positionnement du cathéter de perfusion dans le corps du patient ne fait pas partie de la présente invention. Au contraire, l'invention permet de profiter de la pose ou du changement d'une perfusion déjà installée chez un patient pour effectuer un prélèvement de liquide corporel, tel que du sang, et éviter un nouveau geste intrusif dans le corps du patient.

Selon d'autres modes de réalisation:
● le procédé peut comprendre une étape d'insertion, via le moyen d'insertion, de l'extrémité distale du canal de prélèvement dans la structure tubulaire du dispositif de prélèvement, de telle sorte que ladite extrémité distale soit dirigée vers le cathéter de perfusion, dans le sens d'écoulement du produit de perfusion ;
● le dispositif de prélèvement peut être agencé pour que, en utilisation, la zone d'intubation soit disposée à une distance verticale maximale déterminée de l'extrémité proximale du cathéter ;
● la distance verticale maximale déterminée peut être comprise entre 0 cm et 50 cm ;
● l'extrémité distale du canal de prélèvement peut être agencée à une distance déterminée d'une extrémité proximale du cathéter de perfusion, - ladite distance étant dite « distance d'abouchement », et comprise entre 0 cm et 20 cm, de préférence entre 0 cm et 3 cm ;
● le procédé peut comprendre, en outre, une étape de mise en communication fluidique du réservoir de prélèvement avec un moyen d'analyse du liquide corporel prélevé, et une étape d'analyse du liquide corporel prélevé ;
● le procédé peut comprendre, en outre, une étape d'analyse et de comparaison d'un échantillon de produit déterminé et du liquide corporel prélevé ;
● le procédé peut comprendre, en outre, une étape de génération d'une information de comparaison du liquide corporel prélevé et de l'échantillon de produit déterminé ;
● le procédé peut comprendre, en outre, une étape de mise en communication fluidique d'une deuxième perfusion de produit déterminé avec le circuit fluidique de la première perfusion ;
● le procédé peut comprendre, en outre, une étape de transmission, à un moyen de commande d'écoulement du produit de la deuxième perfusion, de l'information de comparaison du liquide corporel prélevé et de l'échantillon de produit déterminé ;
● le procédé peut comprendre, en outre, une étape de d'affichage d'une information de comparaison du liquide corporel prélevé et de l'échantillon de produit déterminé ;
● le procédé peut comprendre, en outre, une étape d'autorisation de l'écoulement du produit de la deuxième perfusion si l'information de comparaison indique que le liquide corporel et le produit de la deuxième perfusion sont compatibles, et de blocage de l'écoulement du produit de la deuxième perfusion si l'information de comparaison indique que le liquide corporel et le produit de la deuxième la perfusion ne sont pas compatibles ; et/ou
● le procédé peut comprendre, en outre, une étape de génération automatique de l'écoulement du produit de la deuxième perfusion si l'information de comparaison indique que le liquide corporel et le produit de la deuxième la perfusion sont compatibles, et de blocage de l'écoulement du produit de la deuxième perfusion si l'information de comparaison indique que le liquide corporel et le produit de la deuxième la perfusion ne sont pas compatibles.

D'autres caractéristiques de l'invention seront énoncées dans la description détaillée ci-après, faite en référence aux figures annexées qui représentent, respectivement :
- la figure 1, une vue schématique en plan d'un premier mode de réalisation d'un dispositif de prélèvement selon l'invention, installé d'une première manière dans le circuit fluidique d'une perfusion d'un patient ;
- la figure 2, un agrandissement partiel de la figure 1 illustrant, en détail, le dispositif de prélèvement selon l'invention ;
- la figure 3, une vue schématique en plan d'un deuxième mode de réalisation d'un dispositif de prélèvement selon l'invention installé dans le circuit fluidique d'une perfusion d'un patient ;
- la figure 4, un agrandissement partiel de la figure 3 illustrant, en détail, le dispositif de prélèvement selon l'invention ;
- la figure 5, une vue schématique en plan du premier mode de réalisation d'un dispositif de prélèvement selon l'invention installé d'une seconde manière dans le circuit fluidique d'une perfusion d'un patient ;
- la figure 6, un agrandissement partiel de la figure 3 illustrant, en détail, le dispositif de prélèvement selon la figure 5 ;
- les figures 7 et 8, des vues schématiques en plan de deux modes de réalisation d'un cathéter de perfusion selon l'invention ;
- les figures 9 et 10, des vues schématiques en plan de deux modes de réalisation d'une tubulure de perfusion selon l'invention ;
- la figure 11, une vue schématique en plan d'un autre mode de réalisation d'un dispositif de prélèvement selon l'invention, dans lequel la structure tubulaire inclut une partie du canal de prélèvement ;
- les figures 12 et 13, des vues schématiques en coupe du mode de réalisation de la figure 11 ;
- la figure 14, une vue schématique partielle en plan- d'un mode de réalisation du canal de prélèvement;
- les figures 15 et 16, des vues schématiques de deux modes de réalisation d'un réservoir de prélèvement selon l'invention ;
- la figure 17, une vue schématique en plan d'un premier mode de réalisation d'une utilisation particulière du dispositif de prélèvement selon l'invention ;
- la figure 18, une vue schématique en plan d'un deuxième mode de réalisation d'une utilisation particulière du dispositif de prélèvement selon l'invention ; et
- la figure 19, une vue schématique en plan d'un troisième mode de réalisation d'une utilisation particulière du dispositif de prélèvement selon l'invention.

La présente description se rapporte au prélèvement de sang. Cependant, tout liquide corporel pourrait être prélevé en fonction de l'installation préalable de la perfusion sur le patient (urine, liquide céphalorachidien, liquide_pleural, liquide d'ascite, liquide péritonéal, etc.).

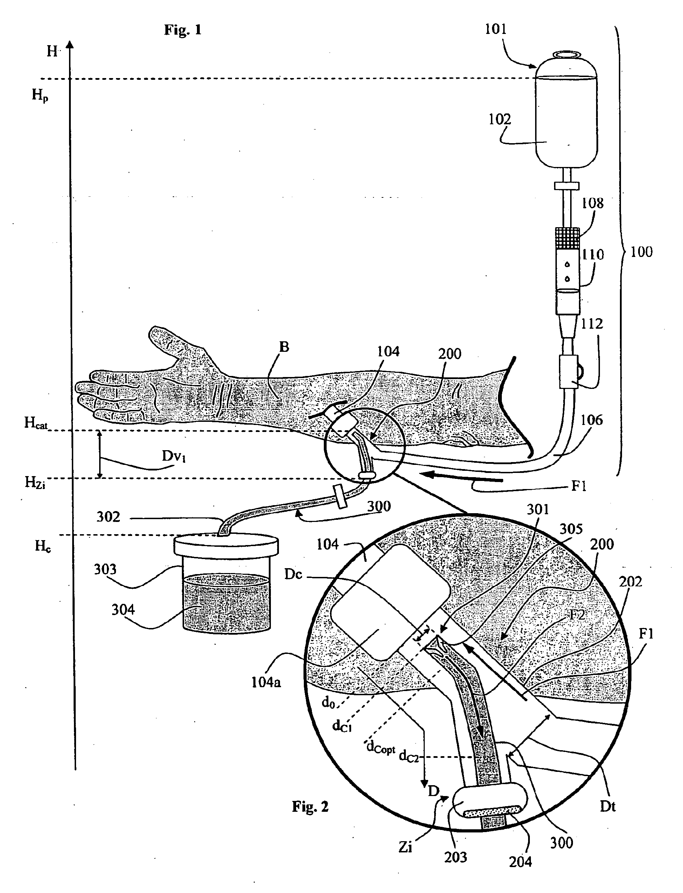

La figure 1, représente le bras B d'un patient équipé d'une perfusion 100. Une telle perfusion est classiquement constituée d'un réservoir 101 de produit de perfusion 102, en communication fluidique avec un cathéter de perfusion 104 par une tubulure 106. Généralement, un filtre 108 et un vase d'expansion 110 sont prévus au sein du circuit fluidique de la perfusion. Une molette 112 de réglage du débit du produit de perfusion 102 est également prévue.

Dans l'ensemble des structures décrites par la suite, on peut prévoir des robinets ou des clamps pour autoriser ou interdire l'écoulement des différents produits.

Le positionnement du cathéter de perfusion dans le corps du patient, et l'installation de la perfusion en général ne font pas partie de la présente invention.

En effet, le dispositif de prélèvement de liquide selon l'invention est destiné à être incorporé, non pas dans un patient, mais dans le circuit fluidique de la perfusion préalablement installée sur un patient, entre le cathéter et le réservoir de perfusion, dans une partie externe du circuit fluidique par rapport au corps du patient.

Le dispositif 200 selon l'invention comprend une structure tubulaire 202 de connexion fluidique au circuit fluidique de la perfusion 100 ; Cette structure tubulaire 202 est munie d'une zone d'intubation Zi, en utilisation, d'un canal de prélèvement 300 comprenant une extrémité distale 301.

Cette structure tubulaire est également munie d'un moyen de maintien 203. Celui-ci est adapté pour permettre, en utilisation, le maintien, dans la structure tubulaire, de la partie du canal de prélèvement 300 comprenant une extrémité distale 301, pour que l'extrémité distale 301 du canal de prélèvement 300 soit dirigée vers le cathéter de perfusion 104, dans le sens d'écoulement F1 du produit de perfusion 102, depuis un réservoir 101 de produit de perfusion vers le-cathéter de perfusion 104.

Le terme « maintien » doit s'entendre au sens large. Il concerne, d'une part, un maintien réversible de la partie du canal de prélèvement insérée dans la structure tubulaire. Dans ce cas, le dispositif selon l'invention ne comprend pas de canal de prélèvement. Celui-ci doit être inséré par l'utilisateur, lors du prélèvement, dans la structure tubulaire (voir figures 1 à 6 et 17 à 19).

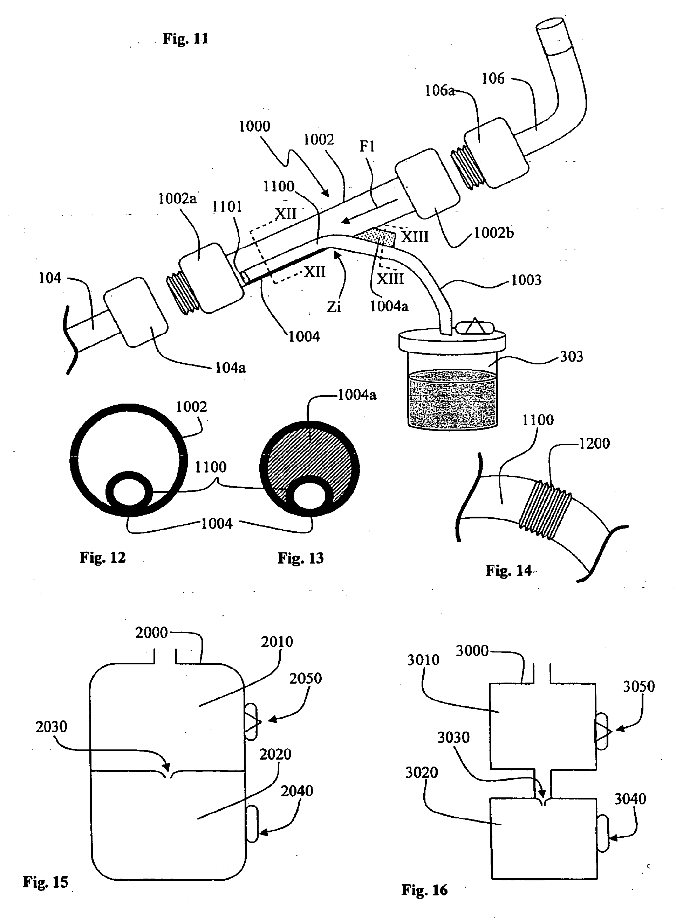

Le terme « maintien » se rapporte également à un maintien définitif dans la structure tubulaire, de la partie du canal de prélèvement pour qu'il ne forme qu'une seule et même pièce comprenant deux tubes. De cette manière, en utilisation, une partie du canal de prélèvement 300 est agencée dans la structure tubulaire de sorte que l'extrémité distale 301 du canal de prélèvement 300 soit dirigée vers le cathéter de perfusion 104, dans le sens d'écoulement F1 du produit de perfusion 102, depuis un réservoir 101 de produit de perfusion vers le cathéter de perfusion 104 (voir figures 11 à 13).

Dans les modes de réalisation des figures 1 à 6 et 17 à 19, la zone d'intubation Zi comprend un moyen d'insertion 204.

Dans le mode de réalisation de la figure 1, la structure tubulaire 202 est un système de connexion en Y présentant une branche destinée à être en communication fluidique avec la tubulure 106 de la perfusion 100, et une branche munie d'une zone d'intubation Zi portant un moyen de maintien 203 et une membrane 204 pour l'insertion étanche du canal 300. Cette membrane est en un matériau étanche conservant son étanchéité après avoir été percé. Alternativement, la structure de la membrane (épaisseur et/ou rigidité) et son agencement dans le système en Y peuvent être tels que le canal de prélèvement est à la fois inséré et maintenu en position d'utilisation, c'est-à-dire à l'intérieur de la structure tubulaire 202. Dans ce cas, la membrane assure les fonctions d'un moyen de maintien et d'un moyen d'insertion.

Le matériau étanche conservant son étanchéité après avoir été percé est choisi parmi un polymère de silicone, tel que le polydiméthylsiloxane (PDMS), du polyméthylmétacrylate (PMMA), du Polychlorure de vinyle (PVC), et du Tygon® (fabriqué par la société Saint-Gobain), etc.

Le canal de prélèvement 300 comprend une extrémité distale 301, qui présente une rigidité suffisante pour percer la membrane 204, et une extrémité proximale 302 destinée à être en communication fluidique avec un réservoir de prélèvement 303. Dans toute la suite de la présente description, les réservoirs utilisés pour recueillir le prélèvement de liquide présentent, avantageusement, une prise d'air obturable.

Selon une variante non illustrée, le moyen de maintien est un connecteur étanche, par exemple de type luer-lock. Dans ce cas, le canal 300 est équipé d'un connecteur luer-lock correspondant, dans lequel passe, de manière étanche, la partie du canal portant l'extrémité distale 301. L'association des connecteurs luer-lock permet l'insertion du canal de prélèvement dans la structure tubulaire et maintient la partie du canal portant l'extrémité distale 301 dirigée vers le cathéter de perfusion 104, dans le sens d'écoulement F1 du produit de perfusion 102, depuis un réservoir 101 de produit de perfusion vers le cathéter de perfusion 104.

Ainsi, l'extrémité distale 301 du canal de prélèvement 300 reste localisée à l'extérieur du cathéter de perfusion et, donc, à l'extérieur du corps du patient.

Il s'agit donc d'une installation ex *vivo* du canal de prélèvement dans un circuit fluidique d'une perfusion située à l'extérieur du corps du patient. Il ne s'agit pas d'une installation *in vivo,* c'est-à-dire dans le corps du patient.

Le procédé de mise en oeuvre sera décrit plus loin.

Le deuxième mode de réalisation 400 d'un dispositif de prélèvement de liquide selon l'invention, illustré à la figure 2, comprend une structure tubulaire 402 de connexion au circuit fluidique de la perfusion 100 munie d'une zone d'intubation Zi comprenant un moyen d'insertion 404, dans la structure tubulaire 402, d'une partie d'un canal de prélèvement 300.

Dans ce mode de réalisation, le moyen d'insertion 404 est une membrane en un matériau étanche conservant son étanchéité après avoir été percé. Cette membrane 404 peut occuper toute ou partie de la paroi de la structure tubulaire 402. Cette membrane 404 présente une structure (épaisseur et/ou rigidité et/ou matériau et/ou agencement) qui lui permet d'assurer à la fois la fonction de moyen de maintien et la fonction de moyen d'insertion. Autrement dit, le canal de prélèvement 300 est inséré et maintenu en position d'utilisation, c'est-à-dire à l'intérieur de la structure tubulaire 402, l'extrémité distale 301 du canal de prélèvement 300 étant dirigée vers le cathéter de perfusion 104, dans le sens d'écoulement F1 du produit de perfusion, depuis le réservoir 101 de produit de perfusion 102 vers le cathéter de perfusion 104. Alternativement, la structure tubulaire peut comprendre un moyen de maintien indépendant de-la membrane qui ne servirait, alors, qu'à l'insertion.

Le matériau étanche conservant son étanchéité après avoir été percé est choisi parmi un polymère de silicone, tel que le polydiméthylsiloxane (PDMS), du polyméthylmétacrylate (PMMA), du Polychlorure de vinyle (PVC), du Tygon® (fabriqué par la société Saint-Gobain), etc.

La réalisation du prélèvement de liquide corporel, illustrée aux figures 1 à 6 et 17 à 19, se fait de la manière suivante.

Lorsqu'un prélèvement de liquide corporel est nécessaire, ici du sang, on incorpore le dispositif de prélèvement 200-400 selon l'invention dans un circuit fluidique d'une perfusion 100 préalablement installée sur un patient B.

Cette incorporation se fait à proximité de l'extrémité proximale 104a du cathéter de perfusion 104 et ne se fait donc pas directement dans le patient, mais dans une partie externe du circuit fluidique par rapport au corps du patient.

Il n'est donc pas nécessaire de réaliser une piqûre supplémentaire au patient. Aucune intervention chirurgicale directe sur le patient n'est nécessaire pour la mise en place du dispositif selon l'invention, ni pour sa mise en oeuvre.

Dans un deuxième temps, on insère, via le moyen d'insertion 204-404 du dispositif de prélèvement 200-400, l'extrémité distale 301 du canal de prélèvement 300 dans la structure tubulaire 202-402 du dispositif de prélèvement, de telle sorte que ladite extrémité distale 301 soit dirigée vers le cathéter de perfusion 104, dans le sens d'écoulement F1 du produit de perfusion. L'extrémité distale 301 du canal de prélèvement 300 reste localisée à l'extérieur du cathéter de perfusion 104 et, donc, à l'extérieur du patient.

Ensuite, on clampe la tubulure de perfusion 106, puis on déclampe le canal de prélèvement 300 et on amène le réservoir de prélèvement 303 à une hauteur H_{c} inférieure à la hauteur H_{cat} du cathéter du patient. Avantageusement, on met le canal de prélèvement et le réservoir à la pression atmosphérique grâce à la prise d'air obturable du réservoir.

De manière surprenante, ceci permet au sang de s'écouler dans le canal de prélèvement 300, à contre-courant selon le sens de la flèche F2, par rapport au produit de perfusion qui est situé entre le cathéter et le clamp de la tubulure 106. Le produit de perfusion situé au dessus du canal de prélèvement et en dessous du clamp de la tubulure 106 n'est pas prélevé en même temps que le sang.

En outre, on s'est aperçu qu'il n'est pas nécessaire d'abaisser le réservoir de perfusion 101 pour que le sang s'écoule dans le canal de prélèvement.

Lorsque le sang s'écoule dans le canal de prélèvement 300, on maintient le réservoir de prélèvement 303 dans cette position pendant une durée suffisante pour obtenir, dans le réservoir de prélèvement 303, le volume de prélèvement 304 souhaité.

Pour obtenir un écoulement du sang dans de bonnes conditions (de vitesse d'écoulement, de confort du patient, etc.), la zone d'intubation Zi du dispositif de prélèvement est agencée de préférence pour que, en position utilisation, elle soit disposée à une distance verticale maximale déterminée Dvₘₐₓ de l'extrémité proximale du cathéter 104. La direction verticale est la direction du champ de pesanteur.

La distance verticale Dv est définie comme la distance entre la hauteur H_{Zi} de la zone d'intubation Zi et la hauteur H_{cat} du cathéter de perfusion sur l'échelle de hauteur H.

La distance verticale maximale Dvₘₐₓ est inférieure à 50 cm. De préférence, elle est comprise entre -50 et 50 cm (le signe négatif signifie que la zone d'intubation est en dessous de l'extrémité proximale du cathéter).

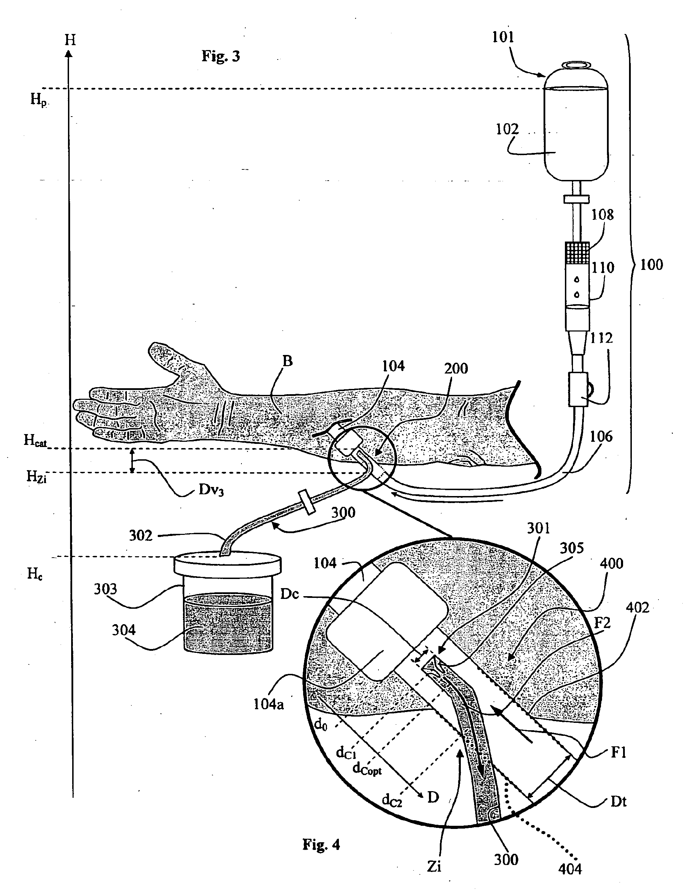

Sur les figures 1 et 3, la zone d'intubation Zi est située sous l'extrémité du cathéter de perfusion 104. La distance verticale, notée respectivement Dv1 et Dv2, est donc négative. Cette disposition est optimale pour obtenir l'écoulement sanguin.

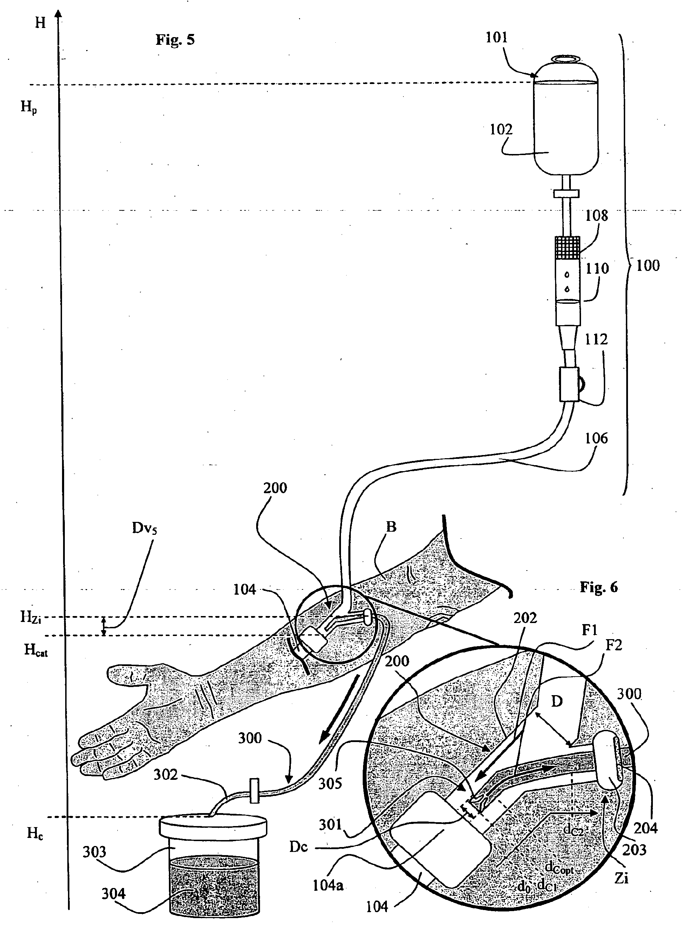

Cependant, dans la pratique, il peut arriver que le cathéter de perfusion 104 soit situé sous la zone d'intubation Zi. Cette situation, illustrée en figure 5, peut arriver, par exemple lorsque le dispositif de perfusion selon l'invention est disposé dans la boucle de sécurité ou dans une tubulure intermédiaire avec une-zone de connexion multiple. Dans ce cas, la distance verticale Dv5 est positive.

Pour obtenir un écoulement du sang, la distance Dv5 doit rester inférieure à la distance verticale maximale déterminée Dvₘₐₓ.

Dans la pratique, et de manière générale, le dispositif de prélèvement est incorporé dans un circuit fluidique de la perfusion à proximité du cathéter de perfusion, c'est-à-dire de telle sorte que la zone d'intubation soit disposée à une distance comprise entre 0 et 50 cm, de préférence entre 5 cm et 15 cm.

De cette manière, lors de l'utilisation, la zone d'intubation a peu de risque de se trouver au dessus de l'extrémité proximale du cathéter, à un distance verticale supérieure à la distance verticale maximale déterminée Dvₘₐₓ.

Au tout début du prélèvement (régime transitoire), le liquide prélevé est constitué par un mélange de sang et du produit de perfusion situé entre le système vasculaire du patient et l'extrémité distale 301 du canal de prélèvement 300.

Il peut donc être préférable de ne connecter le réservoir de prélèvement 303 que lorsque le sang paraît pur (visuellement ou par tout moyen d'analyse).

Alternativement, on peut connaitre le volume de produit de perfusion entre le système vasculaire du patient et l'extrémité distale 301 du canal de prélèvement 300 grâce aux dimensions du cathéter et à la mesure de la distance d_{C1} dite « distance d'abouchement » entre l'extrémité proximale 104a du cathéter 104 (déterminant l'origine d₀ du repère de distance D sur les figures 1 et 2) et l'extrémité distale 301 du canal 300. On peut donc calculer le taux de dilution du sang prélevé dans le réservoir 303.

Selon un autre mode de réalisation, on peut prévoir un réservoir 2000 à deux compartiments 2010 et 2020 séparés par un système anti-retour, tel qu'une valve 2030, un clapet, une bille, un flotteur etc. (voir figure 15). Selon une variante, les deux compartiments 3010 et 3020 du réservoir 3000 sont montés en série de manière indépendante et séparés par un système anti retour 3030 (voir figure 16). Dans les deux exemples illustrés, le compartiment 2020 ou le compartiment 3020 le plus en aval doit permettre le stockage d'une quantité de liquide corporel égale, ou légèrement supérieure, au volume de produit de perfusion situé entre le système vasculaire du patient et l'extrémité distale 301 du canal de prélèvement 300. Au cours de ce stockage, l'air est évacué par une valve d'évacuation d'air 2040 - 3040.

Lorsque le compartiment 2020 ou le compartiment 3020 le plus en aval est rempli, le compartiment 2010 ou le compartiment 3010 amont se remplit. Les systèmes anti retour 2030-3030 évitent que ce liquide dilué, situé dans le compartiment le plus en aval, ne se mélange au liquide « pur » qui est stocké dans le compartiment amont.

Une analyse ultérieure pourra donc être préférentiellement menée sur le liquide pur. A cette fin, les réservoirs des figures 15 et 16 présentent une valve 2050-3050 d'évacuation d'air et de connexion à un moyen d'analyse du liquide corporel prélevé (non représenté sur ces figures).

L'extrémité distale 301 du canal de prélèvement 300 est insérée à une distance d'abouchement déterminée d'une extrémité proximale du cathéter de perfusion.

Afin de connaître cette distance et optimiser le prélèvement, la structure tubulaire du dispositif de prélèvement selon l'invention présente, de préférence, une graduation exprimée en distance.

Avantageusement, cette graduation peut être directement exprimée en volume compris depuis l'extrémité distale du cathéter en contact avec le système vasculaire du patient. Ceci permet un calcul facilité du taux de dilution du prélèvement.

Expérimentalement, on a déterminé que la distance d'abouchement optimale d_{Copt}, est comprise entre 0 et 20 cm, de préférence entre 0 et 3 cm.

Ainsi, à une distance d'abouchement d_{C2}, le prélèvement est de mauvaise qualité : il est trop long ou il-comprend du produit de perfusion.

La distance d'abouchement, le diamètre Dc et la longueur du canal de prélèvement sont adaptés pour permettre un prélèvement de qualité optimale, tout en s'assurant que la durée de prélèvement n'excède pas une minute environ, de préférence dix secondes. Cette durée est considérée, dans la pratique, comme la durée maximale confortable pour le patient.

Dans les modes de réalisation des figures 1 à 6 et 17 à 19, lorsque la quantité prélevée est suffisante, on peut désactiver le moyen de maintien, retirer le canal de prélèvement hors de la structure tubulaire et fermer de manière étanche le moyen d'insertion (automatiquement ou manuellement).

Avantageusement, pour éviter, par exemple lors d'une mauvaise manipulation, un retour de sang depuis le canal de prélèvement vers le circuit fluidique de la perfusion, l'extrémité distale 301 du canal de prélèvement 300 est munie d'un moyen anti retour 305 tel qu'une valve anti retour de type fente de canard ou tricuspide. Alternativement, le moyen anti-retour peut également être situé à l'extrémité proximale du canal de prélèvement 300.

Le dispositif de prélèvement selon l'invention peut être incorporé dans le circuit fluidique de la perfusion entre le cathéter de perfusion et la tubulure de perfusion. Pour cela, le dispositif de prélèvement comprend :
- un moyen de connexion à une extrémité proximale du cathéter de perfusion, et
- un moyen de connexion à l'extrémité distale de la tubulure de perfusion.

Pour installer ce dispositif, l'opérateur connecte le dispositif à l'extrémité distale de la tubulure de perfusion, purge d'air l'ensemble de la tubulure et connecte le dispositif à l'extrémité proximale du cathéter de perfusion.

Les moyens de connexion peuvent être du type luer-lock.

Il peut être envisagé de prévoir un cathéter ou une tubulure de perfusion pré-équipés, lors de leur fabrication, d'un dispositif de prélèvement selon l'invention.

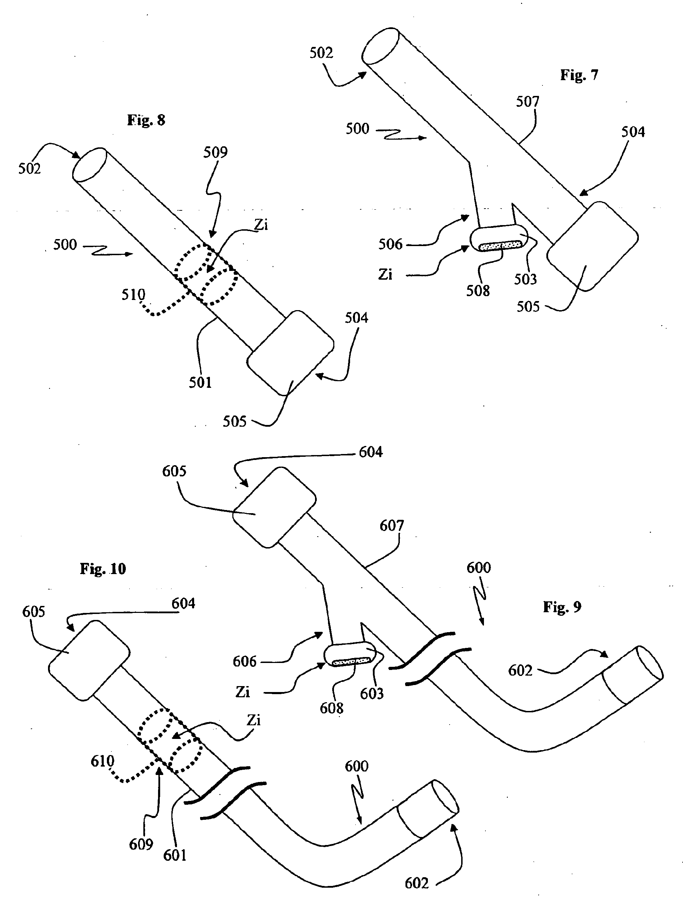

Ainsi, comme le montrent les figures 7 et 8, un cathéter de perfusion 500 selon l'invention comprend :
- une extrémité distale 502 destinée à être insérée dans un patient ;
- une extrémité proximale 504 destinée à être connectée, par exemple par un connecteur luer-lock 505, avec une extrémité distale d'une tubulure de perfusion ; et
- entre l'extrémité distale 502 et l'extrémité proximale 504, un dispositif 506-509 de prélèvement de liquide corporel selon l'invention, agencé pour que, en utilisation, la zone d'intubation Zi soit disposée à une distance verticale maximale déterminée Dvₘₐₓ de l'extrémité proximale du cathéter.

Cet agencement permet un bon écoulement du liquide corporel lors de l'utilisation. La distance verticale maximale déterminée Dvₘₐₓ est comprise entre 0 cm et 50 cm.

Dans ces figures, l'aiguille de perforation du cathéter n'a pas été représentée.

Dans le mode de réalisation de la figure 7, le dispositif 506 présente une structure tubulaire 507 qui est un système de connexion en Y comprenant un moyen de maintien 503 muni d'une membrane d'insertion 508 en un matériau étanche conservant son étanchéité après avoir été percé. Cette structure tubulaire est semblable à celle qui a été décrite en relation avec les figures 1 et 2.

Dans le mode de réalisation de la figure 8, le dispositif 509 présente une structure tubulaire 501 muni d'une membrane 510 en un matériau. étanche conservant son étanchéité après avoir été percé. Cette membrane 510 présente une structure (épaisseur et/ou rigidité et/ou matériau et/ou agencement) qui lui permet d'assurer à la fois le rôle de moyen de maintien et le rôle de moyen d'insertion. Cette membrane 510 est semblable à celle qui a été décrite en relation avec les figures 3 et 4.

Ainsi, lorsque la perfusion est mise en place avec ce cathéter selon l'invention, le dispositif de prélèvement selon l'invention est incorporé dans le circuit fluidique de la perfusion entre le cathéter de perfusion et la tubulure de perfusion.

De même, comme le montrent les figures 9 et 10, une tubulure de perfusion 600 selon l'invention comprend :
- une extrémité proximale 602 destinée à être connectée à un réservoir d'un premier produit de perfusion ;
- une extrémité distale 604 destinée à être connectée, par exemple par un connecteur luer-lock 605, avec une extrémité proximale d'un cathéter de perfusion insérée dans un patient ; et
- entre l'extrémité proximale 602 et l'extrémité distale 604, un dispositif 606-609 de prélèvement de liquide corporel selon l'invention, agencé pour que, en utilisation, la zone d'intubation soit disposée à une distance verticale maximale déterminée Dvₘₐₓ de l'extrémité proximale du cathéter de perfusion.

Ce dispositif de prélèvement est, de préférence, agencé au plus proche de l'extrémité distale 604, pour que, en position d'utilisation, la zone d'intubation soit disposée à une distance comprise entre 0 et 50 cm, de préférence entre 5 et 15 cm.

Dans le mode de réalisation de la figure 9, le dispositif 606 présente une structure tubulaire 607 qui est un système de connexion en Y comprenant un moyen de maintien 603 muni d'une membrane d'insertion 608 en un matériau étanche conservant son étanchéité après avoir été percé. Cette structure tubulaire est semblable à celle qui a été décrite en relation avec la figure 1.

Dans le mode de réalisation de la figure 10, le dispositif 609 présente une structure tubulaire 601 muni d'une membrane 610 en un matériau étanche conservant son étanchéité après avoir été percé. Cette membrane 610 présente une structure (épaisseur et/ou rigidité et/ou matériau et/ou agencement) qui lui permet d'assurer à la fois le rôle de moyen de maintien et le rôle de moyen d'insertion. Cette membrane 610 est semblable à celle qui a été décrite en relation avec la figure 3.

Ainsi, lorsque la perfusion est mise en place, avec cette tubulure selon l'invention, le dispositif de prélèvement selon l'invention est incorporé dans le circuit fluidique de la perfusion entre le cathéter de perfusion et la tubulure de perfusion.

Avec un cathéter ou une tubulure intégrant, dès la fabrication, un dispositif de prélèvement selon l'invention, il n'est pas nécessaire d'arrêter temporairement la perfusion pour incorporer le dispositif de prélèvement selon l'invention.

La mise en oeuvre du procédé décrit précédemment peut être réalisée avec un kit de prélèvement d'un liquide corporel comprenant un dispositif de prélèvement illustré aux figures 1 à 6, et un canal de prélèvement 300 présentant une extrémité distale et une extrémité proximale destinée à être reliée à un réservoir de prélèvement. Bien entendu, le kit peut comprendre, en outre, le réservoir de prélèvement.

Dans un premier exemple de réalisation, le kit de prélèvement selon l'invention peut comprendre un canal de prélèvement et un cathéter de perfusion muni du dispositif de prélèvement, comme ceux qui sont représentés aux figures 7 et 8. Grâce à un tel kit, les procédures d'installation de la perfusion ne sont pas modifiées.

Dans un deuxième exemple de réalisation, le kit de prélèvement selon l'invention peut comprendre un canal de prélèvement et une tubulure de perfusion munie du dispositif de prélèvement selon l'invention, comme celles qui sont représentés aux figures 9 et 10. Grâce à un tel kit, les procédures d'installation de la perfusion ne sont pas modifiées.

Un autre mode de réalisation d'un dispositif de prélèvement 1000 selon l'invention est illustré à la figure 11. Il comprend une structure tubulaire 1002 de connexion au circuit fluidique de la perfusion représenté, dans cette figure 11, par un cathéter 104 et une tubulure 106. A cette fin, la structure tubulaire 1002, le cathéter 104 et la tubulure présentent, de préférence, des connexions luer-lock, respectivement 1002a, 1002b, 104a et 106a.

Le dispositif de prélèvement 1000 comprend également un moyen de maintien 1004 adapté pour permettre, en utilisation, le maintien, à l'intérieur de la structure tubulaire 1002, d'une partie d'un canal de prélèvement 1100 comprenant une extrémité distale 1101. De cette manière, en utilisation, l'extrémité distale 1101 du canal de prélèvement 1100 est dirigée vers le cathéter de perfusion 104, dans le sens d'écoulement F1 du produit de perfusion.

La partie du canal de prélèvement 1100 comprenant l'extrémité distale 1101 est donc directement incorporée dans la structure tubulaire 1002, et une partie 1003 du canal de prélèvement 1100 comprenant une extrémité proximale débouche hors la structure tubulaire au niveau de la zone d'intubation Zi.

Le moyen de maintien 1004 est ici constitué par la liaison mécanique entre le canal 1100 et la structure tubulaire. Comme le montrent les figures 12 et 13 qui sont des coupes transversales de la figure 11, le canal 1100 et la structure tubulaires 1002 constituent une seule et même pièce. Le moyen de maintien peut être une soudure longitudinale.

L'extrémité distale du canal de prélèvement est agencée à une distance d'abouchement déterminée de l'extrémité proximale du cathéter de perfusion, comprise entre 0 cm et 20 cm, de préférence entre 0 cm et 3 cm. Cette distance n'est pas réglable par le personnel soignant, mais prédéfinie lors de la fabrication du dispositif. Ceci permet une manipulation facilitée en évitant un geste technique supplémentaire au personnel soignant.

Dans la figure 11, le moyen de maintien comprend, de manière optionnelle, une partie massive 1004a ressemblant à la branche en Y des modes de réalisation des figures 1 et 2. Cette partie massive permet de soutenir partiellement la partie 1003 débouchant de la partie tubulaire 1002 et, ainsi, éviter la plicature du canal 1100. Cette partie massive peut se situer au dessus, au dessous ou tout autour du canal de prélèvement.

Un dispositif de prélèvement selon l'invention incorporant directement une partie du canal de prélèvement évite à l'utilisateur d'avoir à insérer le canal dans la structure tubulaire. En effet, cette insertion est un geste médical qui peut ne pas être fait de manière satisfaisante, par exemple si la distance d'abouchement est trop importante. En outre, l'insertion peut engendrer une plicature du canal qui empêche l'écoulement du liquide corporel vers le réservoir 303.

Pour éviter ce phénomène de plicature, le canal de prélèvement 1100 peut comprendre au moins une structure en accordéon 1200 (voir figure 14) lui permettent de se courber sans formation de plis.

Cette structure en accordéon 1200 peut équiper le canal de prélèvement de tous les modes de réalisation du dispositif selon l'invention.

Dans tous les dispositifs ou kits de prélèvement précédents, le rapport entre le diamètre Dc du canal de prélèvement et le diamètre Dt de la structure tubulaire du dispositif de prélèvement est, de préférence, inférieur à 1, et typiquement compris entre 1/20 et 1/3. Ceci permet d'assurer la continuité de l'apport en produit de perfusion malgré le prélèvement sanguin.

De même, le diamètre externe du cathéter de perfusion est compris, de préférence, entre 14 G (gauge) et 24 G, avantageusement entre 14 G et 18 G.

Plus ce diamètre est important, plus le débit de prélèvement peut être important. En jouant sur la hauteur du réservoir par rapport au cathéter du patient, on peut régler la vitesse d'écoulement et augmenter le débit. Le prélèvement peut donc se faire en un temps confortable pour le patient, d'une minute ou moins, de préférence dix secondes environ.

De préférence, le canal de prélèvement présente une longueur entre ses deux extrémités comprise entre 10 cm et 100 cm, avantageusement entre 20 cm et 50 cm.

Le dispositif de prélèvement selon l'invention peut avantageusement être utilisé pour réaliser un système de perfusion sécurisé, tel qu'une transfusion sanguine sécurisée.

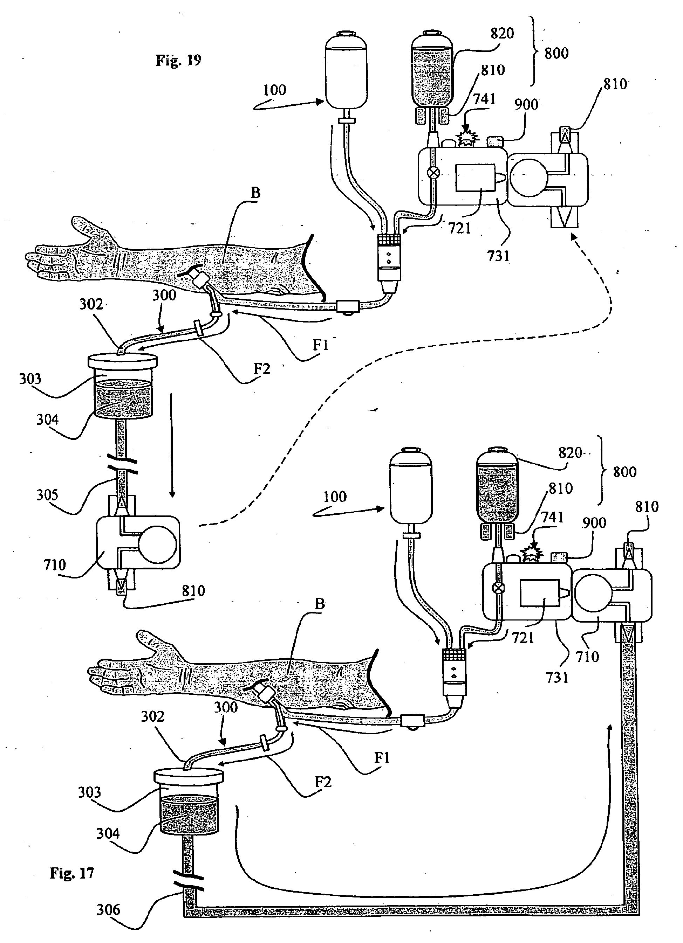

Ce système et sa mise en oeuvre son illustré aux figures 17 à 19 décrites ci-après.

Ainsi, le procédé de mise en oeuvre du dispositif selon l'invention peut comprendre, en outre, une étape de mise en communication fluidique, via une conduite 305, du réservoir de prélèvement 303 avec un moyen d'analyse 700 du liquide corporel prélevé 304, puis une étape d'analyse de ce liquide 304. Ce moyen d'analyse 700 peut être compris dans un kit de prélèvement d'un liquide corporel selon l'invention.

Dans le mode de réalisation de la figure 17, le moyen d'analyse 700 comprend une chambre de réaction 710 et un moyen de détection 720.

La chambre de réaction 710 peut présenter toute forme adaptée à la réaction devant être détectée.

Un exemple de réalisation d'une chambre de réaction est une cassette comprenant une biopuce munie d'un circuit fluidique.

Un exemple particulièrement intéressant, dans le cadre d'un système de transfusion sanguine sécurisée, est l'utilisation de biopuces d'or biofonctionnalisées 710 en combinaison avec un dispositif de détection 720 à base de transductions optiques (tel que ceux commercialisés par la société GE Healthcare sous la dénomination « système BIAcore »).

Basé sur la résonance de plasmon de surface, ce type de dispositif mesure une variation d'angle d'extinction de résonance (dépendante d'une variation d'indice à l'interface or/diélectrique) qui peut être corrélée à une variation de masse.

Réalisée sur un support en verre muni d'une mince couche d'or, la fonctionnalisation se déroule en deux étapes. Une première étape permet la reconstitution d'un film mince organique présentant certaines fonctions chimiques activables (type SH, COOH, NH2....) pour le greffage ultérieur des anticorps. Lors de la seconde étape, cette couche est activée et les anticorps déposés à sa surface sont immobilisés.

Les inventeurs ont découvert qu'après traitement chimique de la surface d'or, l'immobilisation des anticorps anti-A et anti-B de type IgM était significativement améliorée à un pH d'environ 4,65.

Dans ces conditions de pH, le taux de greffage atteint en moyenne 1500 IgM/µm² ce qui permet d'impliquer jusqu'à 100 000 anticorps par globule rouge capturé.

Cela entraîne une forte interaction entre les hématies et l'immunocapteur présentant une surface fonctionnalisée par des IgM anti-A et anti-B, et ce même après plusieurs rinçages

Ainsi, le moyen 700 est capable d'analyser le liquide corporel prélevé 304 en interprétant les informations de détection, issues du moyen de détection 720, concernant les interactions entre les globules du sang et les anticorps présents sur la biopuce.

Cette interaction est très sensible et permet une analyse de compatibilité sanguine même en cas de pathologie ou d'antigènes faibles.

D'autres types d'interaction et d'autres moyens de détection que ceux précédemment décrit peuvent être utilisé pour analyser le sang (détection de virus, de protéines, de cellules rares circulantes, etc.) ou d'autres liquides corporels prélevés.

Le procédé selon l'invention prévoit également une étape d'analyse d'un échantillon 810 de produit déterminé et une étape de comparaison du liquide corporel prélevé 304 et de l'échantillon 810 de produit déterminé.

Par exemple, dans le cas d'une transfusion sanguine, le produit déterminé est du sang stocké dans un des réservoirs secondaires 810 d'échantillonnage, séparables du réservoir principal 820 de la poche de transfusion 800.

De préférence, le moyen d'analyse 700 est également apte à analyser l'échantillon de produit déterminé 810 et à le comparer avec le liquide corporel prélevé 304.

Le procédé selon l'invention prévoit également une étape d'affichage de l'information de comparaison du liquide corporel prélevé et de l'échantillon de produit déterminé. A cette fin, le kit de prélèvement selon l'invention comprend un moyen d'affichage de l'information de comparaison du liquide corporel prélevé et de l'échantillon de produit déterminé.

Dans le mode de réalisation illustré en figure 17, le réservoir de prélèvement 303 est en communication fluidique, via une conduite 306, avec une chambre de réaction 712.

La conduite 306 est suffisamment longue pour permettre à un opérateur, lorsque la chambre de réaction est prête pour l'analyse et la comparaison, de placer la chambre 710 sur un moyen de fixation (non représenté) à un moyen de commande d'écoulement 731 semblable à celui décrit en figure 12. Celui-ci est muni d'un moyen de détection et d'analyse 721 et d'un moyen d'affichage 741 de l'information de comparaison I_{C} (non représentée) émise par le moyen 721.

Le moyen de fixation peut être une pince prenant en sandwich la chambre de réaction 710, de telle sorte que la position de la chambre est optimale pour la détection et l'analyse par le moyen 721.

Ce mode de réalisation évite de déconnecter la chambre de la conduite 306. Les risques de contact direct entre l'opérateur et le liquide prélevé sont donc diminués.

En outre, la détection et l'analyse se font sur la même ligne de perfusion. Puisqu'il n'y a pas de déconnexion entre la chambre 710 et le patient (via le dispositif de prélèvement 200-400, le canal 300, le réservoir 303 et la conduite 306), il devient impossible de faire une erreur de connexion entre la chambre et le moyen de commande d'écoulement 731 ; il ne peut pas y avoir d'inversion de chambre entre un patient et un autre patient.

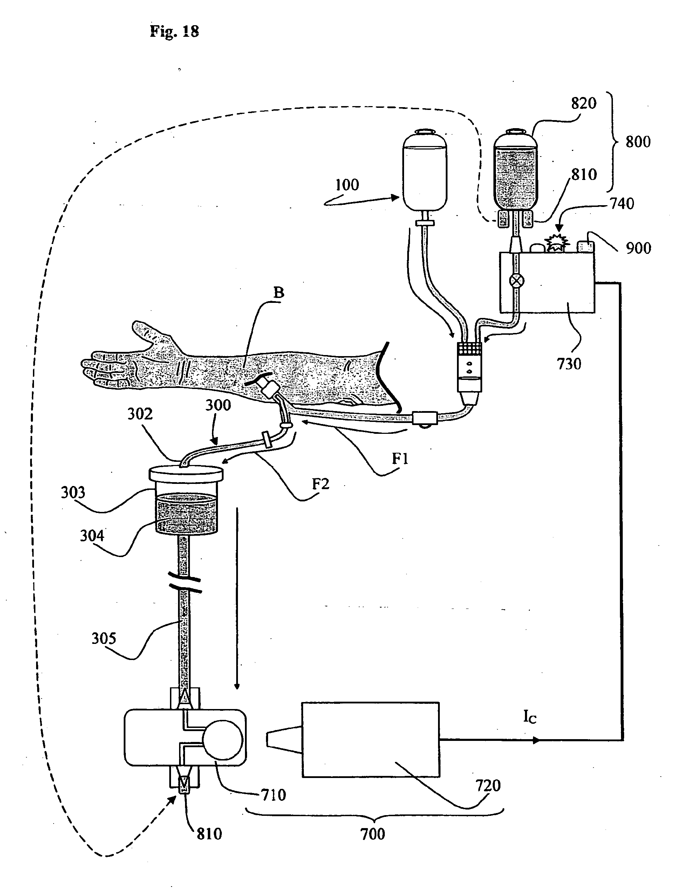

Dans le mode de réalisation illustré en figure 18, le moyen de détection 720 du moyen d'analyse 700, localisé à proximité de la chambre de réaction 710, transmet une information de comparaison I_{C} à un moyen de commande d'écoulement 730 d'une deuxième perfusion (ici un circuit de transfusion sanguine). Le moyen de commande d'écoulement 730 est muni, de préférence, d'un moyen d'affichage 740 de l'information de comparaison.

De manière pratique, le moyen d'analyse 700 est, dans ce mode de réalisation, localisé en dessous du cathéter du patient, à proximité du réservoir de prélèvement 303.

Un tel mode de réalisation présente l'avantage de ne nécessiter qu'une faible longueur de conduites fluidiques (canal 300 et conduite 305). Cependant, l'encombrement global du système peut être important puisqu'il comprend un moyen d'analyse 700 et un moyen de commande d'écoulement 730 indépendant.

Dans le mode de réalisation illustré en figure 19, la chambre de réaction 710 est, comme dans la figure 18, connectée au réservoir 303 de liquide prélevé et à un échantillon 810 de produit déterminé.

Lorsque la chambre de réaction est prête pour l'analyse et la comparaison, l'opérateur clampe la conduite 305 (une mollette de fermeture, non représentée, est préférentiellement prévue à cet effet) et connecte la chambre de réaction 710 à un moyen d'analyse et de commande d'écoulement 731. Celui-ci est muni d'un moyen de détection et d'analyse 721 et d'un moyen d'affichage 741 de l'information de comparaison I_{C} (non représentée) émise par le moyen 721. La connexion de la chambre 710 sur le moyen de commande 731 peut être faite à l'aide d'un moyen de fixation (non représenté).

Le moyen de fixation peut être une pince prenant en sandwich la chambre de réaction 710, de telle sorte que la position de la chambre est optimale pour la détection et l'analyse par le moyen 721.

Ce mode de réalisation présente l'avantage d'être plus compact que celui illustré en figure 18. Cependant, il nécessite une étape de déconnexion fluidique de la chambre de réaction 710, ce qui peut constituer un risque de contact entre l'opérateur et le liquide prélevé.

Dans les trois modes de réalisation précédents, lorsque l'analyse est opérée par le moyen d'analyse 700-720-721, celui-ci transmet au moyen de commande d'écoulement 730-731 de la deuxième perfusion, l'information de comparaison I_{C} du liquide corporel prélevé et de l'échantillon de produit déterminé.

Cette information est alors affichée grâce au moyen d'affichage 740-741.

Le personnel soignant sait, alors, si le produit de la deuxième perfusion est compatible avec le patient et/ou la situation médicale qui a été diagnostiquée. Dans l'exemple de la transfusion sanguine, le personnel soignant sait si le sang de la poche de transfusion est d'un groupe ABO compatible avec le groupe ABO du patient.

Dans un autre exemple, le personnel soignant pourrait déterminer si l'antibiotique présent dans la poche de perfusion ne provoque pas de réaction allergique avec le patient.

Lorsque l'information de comparaison indique que le produit de la deuxième transfusion est compatible avec le patient, le personnel soignant peut mettre en communication fluidique la deuxième perfusion de produit avec le circuit fluidique de la première perfusion.

Cette mise en communication fluidique se fait, avantageusement, par l'intermédiaire du moyen de commande de l'écoulement.

Ce mode de réalisation permet de ne connecter la poche de produit de la deuxième perfusion que si elle est compatible avec le patient. Cela évite de gâcher une poche de produit car ce système permet de récupérer-la poche considérée comme incompatible.

Cependant, il est aussi envisageable de prévoir que la poche de la deuxième perfusion soit déjà connectée au moyen de commande lorsque l'analyse et la comparaison sont effectuées.

Ensuite, le personnel soignant active manuellement un moyen de génération 900 de l'écoulement dudit produit.

Selon un mode de réalisation préféré, le procédé selon l'invention, comprend une étape de blocage de l'écoulement du produit de la deuxième perfusion si l'information de comparaison indique que le liquide corporel et le produit de la deuxième perfusion ne sont pas compatibles, et d'autorisation de l'écoulement du produit de la deuxième perfusion si l'information de comparaison indique que le liquide corporel et le produit de la deuxième perfusion sont compatibles.

De cette manière, si le personnel soignant prend, par mégarde, la décision de générer manuellement l'écoulement en activant le moyen de génération 900, le produit de la deuxième perfusion ne s'écoule pas.

A cette fin, le moyen de commande d'écoulement comprend une électrovanne en communication fluidique avec le circuit de la deuxième perfusion.

Il est également envisageable, pour certaines applications, que le moyen d'analyse soit aussi apte à contrôler le moyen de commande d'écoulement pour qu'il génère automatiquement l'écoulement du produit de la deuxième perfusion si le liquide corporel et le produit de la deuxième perfusion sont compatibles, et pour qu'il ne génère pas l'écoulement du produit de la deuxième perfusion si le liquide corporel et le produit de la deuxième perfusion ne sont pas compatibles.

Selon un autre mode de réalisation non illustré, le dispositif de prélèvement comprend un réservoir, avantageusement muni d'une prise d'air obturable, relié à une tubulure connectable à son extrémité distale par une liaison de type tuer-tock. Ce système peut être mis en place sur un robinet trois voies déjà en place, disposé sur un prolongateur ou sur une rampe de robinet connecté d'une part au cathéter en place sur le patient et d'autre part à la tubulure de perfusion. Le prélèvement est alors réalisé en abaissant le réservoir, comme décrit précédemment. Ce mode de réalisation présente l'avantage d'être très simple. Néanmoins, il entraine un volume dilué (sang et liquide de perfusion) et un temps de remplissage plus importants qu'avec les modes de réalisation décrits précédemment.

L'invention permet donc un contrôle ultime sur la même ligne de perfusion connectée au patient. Il n'y a donc plus d'aléas entre l'analyse pour le contrôle ultime et la mise en oeuvre effective du traitement lui-même puisque le contrôle et l'autorisation de traitement sont liés directement au patient, sans intervention potentiellement erronée du personnel soignant.

Le procédé selon l'invention n'a pas pour objet de diagnostiquer la situation médicale (maladie, accident, hémorragie, etc.) et/ou de choisir le traitement *ad hoc* puisque ces étapes ont préalablement été effectuées par le médecin, en fonction de la situation médicale et du patient.

Le procédé vise à s'assurer que le traitement qui va être prodigué est bien conforme au choix du médecin. Par exemple, le médecin a diagnostiqué la situation médicale X et il a choisi de traiter le patient Y avec le produit Z qui est compatible avec la situation médicale X et le patient Y. Après ce diagnostic, le procédé selon l'invention permet d'effectuer un contrôle ultime lors de la mise en oeuvre du traitement, en vérifiant que c'est bien le produit Z, compatible avec la situation médicale X et le patient Y, qui est appliqué.

Pour l'exemple du sang, le procédé selon l'invention permet d'effectuer un contrôle ultime lors de la mise en oeuvre de la transfusion, en vérifiant que le sang de la poche de transfusion, nécessaire au traitement de la situation médicale X préalablement diagnostiquée, est compatible avec le groupe ABO du sang du patient Y.

Ce procédé ne vise donc pas à diagnostiquer une situation médicale nécessitant une transfusion sanguine ou à éviter une situation médicale nécessitant une transfusion sanguine.

C'est une méthode visant à éviter un accident médical (incompatibilité entre le produit de traitement et le patient) et non visant à traiter un état pathologique.

## Revendications

1. Dispositif de prélèvement (200, 400, 506, 509, 606, 609, 1000) d'un liquide corporel, tel que du sang, destiné à être incorporé dans un circuit fluidique d'une perfusion (100) d'un patient muni d'un cathéter de perfusion (104, 504), et comprenant une structure tubulaire (202, 402, 501, 507, 601, 607, 1002) de connexion au circuit fluidique de la perfusion munie :
- d'une zone d'intubation (Zi), en utilisation, d'un canal de prélèvement (300, 1100) comprenant une extrémité distale (301) ; et
- d'un moyen de maintien (203, 503, 603, 1004, 1004a), en utilisation, dans la structure tubulaire, de la partie du canal de prélèvement (300, 1100) comprenant une extrémité distale (301, 1101), de sorte que ladite extrémité distale (301, 1101) soit dirigée vers le cathéter de perfusion (104), dans le sens d'écoulement (F1) du produit de perfusion (102), depuis un réservoir (101) de produit de perfusion vers le cathéter de perfusion (104).

2. Dispositif de prélèvement (1000) selon la revendication 1, dans lequel la partie comprenant l'extrémité distale (1101) du canal de prélèvement (1100) est fixée dans la structure tubulaire (1002), et une partie (1003) comprenant une extrémité proximale du canal de prélèvement (1100) débouche hors la structure tubulaire (1002) au niveau de la zone d'intubation (Zi).

3. Dispositif de prélèvement 200, 400, 506, 509, 606, 609) selon la revendication 1, dans lequel la zone d'intubation comprend un moyen d'insertion (204, 404) adapté pour permettre, en utilisation, l'insertion, dans la structure tubulaire, de la partie du canal de prélèvement (300) comprenant l'extrémité distale (301).

4. Dispositif de prélèvement selon l'une quelconque des revendications 1 à 3, dans lequel la zone d'intubation est agencée pour que, en utilisation, elle soit disposée à une distance verticale maximale déterminée (Dvₘₐₓ) de l'extrémité proximale du cathéter.

5. Dispositif de prélèvement selon la revendication 4, dans lequel la distance verticale maximale déterminée (Dvₘₐₓ) est comprise entre 0 cm et 50 cm.

6. Dispositif de prélèvement selon l'une quelconque des revendications 3 à 5, dans lequel le moyen d'insertion est choisi parmi :
- une membrane (404) en un matériau étanche conservant son étanchéité après avoir été percé ; et
- un connecteur étanche.

7. Dispositif de prélèvement selon la revendication 6, dans lequel le matériau étanche conservant son étanchéité après avoir été percé est choisi parmi un polymère de silicone, tel que le polydiméthylsiloxane (PDMS), du polyméthylmétacrylate (PMMA), du Polychlorure de vinyle (PVC), et du Tygon®.

8. Dispositif de prélèvement selon l'une quelconque des revendications 1 à 7, comprenant, en outre :
- un moyen de connexion à une extrémité proximale d'un cathéter de perfusion, et
- un moyen de connexion à une extrémité distale d'une tubulure de perfusion.

9. Cathéter de perfusion (500), comprenant :
- une extrémité distale (502) destinée à être insérée dans un patient ;
- une extrémité proximale (504) destinée à être connectée avec une extrémité distale d'une tubulure de perfusion
**caractérisé en ce qu'**il comprend, entre l'extrémité distale (502) et l'extrémité proximale (504), un dispositif de prélèvement (506-509) de liquide corporel selon l'une quelconque des revendications 1 à 8, agencé pour que, en utilisation, la zone d'intubation soit disposée à une distance verticale maximale déterminée (Dvₘₐₓ) de l'extrémité proximale du cathéter.

10. Tubulure de perfusion (600), comprenant :
- une extrémité proximale (602) destinée à être connectée à un réservoir d'un premier produit de perfusion ;
- une extrémité distale (604) destinée à être connectée avec une extrémité proximale d'un cathéter de perfusion insérée dans un patient ;
**caractérisé en ce qu'**elle comprend, entre l'extrémité proximale (602) et l'extrémité distale (604), un dispositif de prélèvement (606-609) de liquide corporel selon l'une quelconque des revendications 1 à 8, agencé pour que, en utilisation, la zone d'intubation soit disposée à une distance verticale maximale déterminée (Dvₘₐₓ) de l'extrémité proximale du cathéter.

11. Kit de prélèvement d'un liquide corporel, **caractérisé en ce qu'**il comprend :
- un canal de prélèvement (300) comprenant une extrémité proximale (302) destinée à être reliée à un réservoir de prélèvement (303), et une extrémité distale (301), et
- un dispositif de prélèvement selon l'une quelconque des revendications 1 à 8.

12. Kit de prélèvement d'un liquide corporel selon la revendication 11, présentant un rapport entre le diamètre intérieur (Dc) du canal de prélèvement et le diamètre extérieur (Dt) de la structure tubulaire du dispositif de prélèvement inférieur à 1, typiquement compris entre 1/20 et 1/3.

13. Kit de prélèvement d'un liquide corporel selon l'une quelconque des revendications 11 ou 12, dans lequel le canal de prélèvement (300) présente une longueur entre ses deux extrémités comprise entre 10 cm et 100 cm, de préférence entre 20 cm et 50 cm.

14. Kit de prélèvement d'un liquide corporel selon l'une quelconque des revendications 11 à 13, comprenant, en outre, un moyen d'analyse (700) du liquide corporel prélevé.

15. Kit de prélèvement d'un liquide corporel selon la revendication 14, dans lequel le moyen d'analyse (700) est également apte à analyser un échantillon de produit déterminé et à comparer le liquide corporel prélevé et l'échantillon de produit déterminé.

16. Kit de prélèvement d'un liquide corporel selon l'une des revendications 14 ou 15, dans lequel le moyen d'analyse (700) comprend une chambre de réaction (710) et un moyen de détection (720, 721).

17. Kit de prélèvement d'un liquide corporel selon l'une quelconque des revendications 14 à 16, comprenant, en outre, un moyen de commande d'écoulement (730, 731) du produit d'une deuxième perfusion, le moyen d'analyse (700, 720) étant apte à transmettre au moyen de commande d'écoulement (730, 731) une information de comparaison (I_{C}) du liquide corporel prélevé (304) et de l'échantillon de produit déterminé (810).

18. Kit de prélèvement d'un liquide corporel selon la revendication 17, comprenant un moyen d'affichage (740, 741) de l'information de comparaison (I_{C}) du liquide corporel prélevé (304) et de l'échantillon de produit déterminé (810).

19. Kit de prélèvement d'un liquide corporel selon l'une quelconque des revendications 17 ou 18, dans lequel le moyen d'analyse (700, 720) est apte à contrôler le moyen de commande d'écoulement (730, 731) pour qu'il bloque l'écoulement du produit de la deuxième perfusion si le liquide corporel et le produit de la deuxième perfusion ne sont pas compatibles, et pour qu'il autorise l'écoulement du produit de la deuxième perfusion si le liquide corporel et le produit de la deuxième perfusion sont compatibles.

20. Kit de prélèvement d'un liquide corporel selon l'une quelconque des revendications 17 ou 18, dans lequel le moyen d'analyse (700, 720) est apte à contrôler le moyen de commande d'écoulement (730, 731) pour qu'il génère automatiquement l'écoulement du produit de la deuxième perfusion si le liquide corporel et le produit de la deuxième perfusion sont compatibles, et pour qu'il ne génère pas l'écoulement du produit de la deuxième perfusion si le liquide corporel et le produit de la deuxième perfusion ne sont pas compatibles.

21. Kit de prélèvement d'un liquide corporel selon l'une quelconque des revendications 11 à 20, comprenant, en outre, un réservoir de prélèvement (303).

22. Kit de prélèvement d'un liquide corporel selon la revendication précédente, dans lequel le réservoir de prélèvement comprend un moyen d'anti-coagulation du sang.

23. Kit de prélèvement d'un liquide corporel selon l'une quelconque des revendications 21 ou 22, dans lequel le réservoir de prélèvement est un réservoir (2000-3000) à deux compartiments (2010-2020, 3010-3020) séparés par un système anti retour (2030, 3030).

24. Kit de prélèvement d'un liquide corporel selon l'une quelconque des revendications 21 à 23, dans lequel le réservoir de prélèvement peut être un réservoir déformable et actionnable mécaniquement.

25. Kit de prélèvement d'un liquide corporel selon l'une quelconque des revendications 21 à 24, dans lequel le réservoir de prélèvement est mis préalablement en dépression sous vide et maintenu dans cet état par un clamp.

26. Kit de prélèvement d'un liquide corporel selon l'une quelconque des revendications 11 à 25, comprenant un cathéter de perfusion (500) selon la revendication 9, muni du dispositif de prélèvement.

27. Kit de prélèvement d'un liquide corporel selon l'une quelconque des revendications 11 à 25, comprenant une tubulure de perfusion (600) selon la revendication 10, munie du dispositif de prélèvement.

28. Procédé de mise en oeuvre d'un kit de prélèvement d'un liquide corporel selon l'une quelconque des revendications 11 à 27, **caractérisé en ce qu'**il comprend les étapes suivantes :
- incorporer le dispositif de prélèvement (200, 400, 500, 600) dans un circuit fluidique d'une perfusion (100) préalablement installée sur un patient muni d'un cathéter de perfusion (104), dans une partie externe du circuit fluidique par rapport au corps du patient (B) ;
- maintenir, à l'intérieur de la structure tubulaire, la partie du canal de prélèvement (300) comprenant l'extrémité distale (301), de sorte que ladite extrémité distale (301) soit dirigée vers le cathéter de perfusion (104), dans le sens d'écoulement (F1) du produit de perfusion (102), depuis un réservoir (101) de produit de perfusion vers le cathéter de perfusion (104) ;
- amener le réservoir de prélèvement (303) à une hauteur (H_{c}) inférieure à celle (H_{cat}) du cathéter du patient, de telle sorte que le liquide corporel s'écoule dans le canal de prélèvement (300), à contre-courant (F2) par rapport au produit de perfusion puis vers le réservoir de prélèvement ; et
- maintenir le réservoir de prélèvement à une hauteur inférieure à celle du cathéter du patient pendant une durée suffisante pour obtenir, dans le réservoir de prélèvement, un volume suffisant pour constituer un prélèvement de liquide corporel.

29. Procédé selon la revendication 28, de mise en oeuvre d'un kit de prélèvement d'un liquide corporel selon l'une quelconque des revendications 9 à 20 comprenant un dispositif de prélèvement selon l'une quelconque des revendications 3 à 8, comprenant une étape d'insertion, via le moyen d'insertion (204, 404), de l'extrémité distale (301) du canal de prélèvement (300) dans la structure tubulaire (202, 402) du dispositif de prélèvement, de telle sorte que ladite extrémité distale soit dirigée vers le cathéter de perfusion (104), dans le sens d'écoulement (F1) du produit de perfusion.

30. Procédé selon l'une des revendications 28 ou 29, dans lequel le dispositif de prélèvement (200, 400, 500, 600) est agencé pour que, en utilisation, la zone d'intubation soit disposée à une distance verticale maximale déterminée (Dvₘₐₓ) de l'extrémité proximale du cathéter.

31. Procédé selon la revendication 30, dans lequel la distance verticale maximale déterminée (Dvₘₐₓ) est comprise entre 0 cm et 50 cm.

32. Procédé selon l'une des revendications 28 à 31, dans lequel l'extrémité distale du canal de prélèvement est agencée à une distance déterminée d'une extrémité proximale du cathéter de perfusion, ladite distance étant dite « distance d'abouchement», et comprise entre 0 cm et 20 cm, de préférence entre 0 cm et 3 cm.

33. Procédé selon l'une quelconque des revendications 28 à 32, comprenant, en outre, une étape de mise en communication fluidique du réservoir de prélèvement avec un moyen d'analyse du liquide corporel prélevé, et une étape d'analyse du liquide corporel prélevé.

34. Procédé selon la revendication 33, comprenant, en outre, une étape d'analyse et de comparaison d'un échantillon de produit déterminé et du liquide corporel prélevé.

35. Procédé selon la revendication 34, comprenant, en outre, une étape de génération d'une information de comparaison du liquide corporel prélevé et de l'échantillon de produit déterminé

36. Procédé selon la revendication 35, comprenant, en outre, une étape de mise en communication fluidique d'une deuxième perfusion de produit déterminé avec le circuit fluidique de la perfusion préalablement installée.

37. Procédé selon la revendication 36, comprenant, en outre, une étape de transmission, à un moyen de commande d'écoulement du produit de la deuxième perfusion, de l'information de comparaison du liquide corporel prélevé et de l'échantillon de produit déterminé.

38. Procédé selon la revendication 37, comprenant, en outre, une étape de d'affichage d'une information de comparaison du liquide corporel prélevé et de l'échantillon de produit déterminé.

39. Procédé selon l'une quelconque des revendications 37 ou 38 comprenant, en outre, une étape d'autorisation de l'écoulement du produit de la deuxième perfusion si l'information de comparaison indique que le liquide corporel et le produit de la deuxième perfusion sont compatibles, et de blocage de l'écoulement du produit de la deuxième perfusion si l'information de comparaison indique que le liquide corporel et le produit de la deuxième la perfusion ne sont pas compatibles.

40. Procédé selon l'une quelconque des revendications 37 ou 38, comprenant, en outre, une étape de génération automatique de l'écoulement du produit de la deuxième perfusion si l'information de comparaison indique que le liquide corporel et le produit de la deuxième la perfusion sont compatibles, et de blocage de l'écoulement du produit de la deuxième perfusion si l'information de comparaison indique que le liquide corporel et le produit de la deuxième la perfusion ne sont pas compatibles.

## Patentansprüche

1. Vorrichtung (200, 400, 506, 509, 606, 609, 1000) zur Entnahme einer Körperflüssigkeit wie etwa Blut, die dazu bestimmt ist, in einen Fluidkreislauf einer Infusion eines Patienten eingegliedert zu sein, der einen Infusionskatheter (104, 504) umfasst, und die eine röhrenartige Struktur (202, 402, 501, 507, 601, 607) zur Verbindung mit dem Fluidkreislauf der Infusion umfasst, mit:
- einer Intubationszone (Zi), in Verwendung, eines Entnahmekanals (300, 1100) mit einem distalen Ende (301); und
- einem Mittel (203, 503, 603, 1004, 1004a) zum Halten, in Verwendung, in der röhrenartigen Struktur, des Teils des Entnahmekanals (300, 1100), der ein distale Ende (301, 1101) umfasst, und zwar derart, dass das distale Ende (301, 1101) zu dem Infusionskatheter (104), in Strömungsrichtung (F1) der Infusionslösung (102) von einem Infusionslösungsbehälter (101) zu dem Infusionskatheter (104), gerichtet ist.

2. Entnahmevorrichtung (1000) nach Anspruch 1, in der der Teil, der das distale Ende (1101) des Entnahmekanals (1100) umfasst, in der röhrenartige Struktur (1002) befestigt ist und ein Teil (1003), der ein proximales Ende des Entnahmekanals (1100) umfasst, auf Höhe der Intubationszone (Zi), außerhalb der röhrenartige Struktur (1002) mündet.

3. Entnahmevorrichtung (200, 400, 506, 509, 606, 609) nach Anspruch 1, in der die Intubationszone ein Einführungsmittel (204, 404), um, in Verwendung, ein Einführen des Teil des Entnahmekanals (300) in die röhrenartige Struktur zu ermöglichen, der das distale Ende (301) umfasst.

4. Entnahmevorrichtung nach einem der Ansprüche 1 bis 3, in der die Intubationszone so ausgelegt ist, dass sie, in Verwendung, in einem bestimmten maximalen vertikalen Abstand (Dvₘₐₓ) von dem proximalen Ende des Katheters angeordnet ist.

5. Entnahmevorrichtung nach Anspruch 4, in der der bestimmte maximale vertikale Abstand (Dvₘₐₓ) zwischen 0 cm und 50 cm beträgt.

6. Entnahmevorrichtung nach einem der Ansprüche 3 bis 5, in der das Einführungsmittel ausgewählt ist aus:
- einer Membran (404) aus einem dichten Material, das seine Dichtheit bewahrt, nachdem es durchstochen worden ist; und
- einem dichten Verbinder.

7. Entnahmevorrichtung nach Anspruch 6, in der das dichte Material, das seine Dichtheit bewahrt, nachdem es durchstochen worden ist, ausgewählt ist aus einem Silikonpolymer wie etwa Polydimethylsiloxan (PDMS), Polymethylmetacrylat (PMMA), Polyvinylchlorid (PVC) und Tygon®.

8. Entnahmevorrichtung nach einem der Ansprüche 1 bis 7, die ferner umfasst:
- ein Mittel zum Verbinden mit einem proximalen Ende eines Infusionskatheters, und
- ein Mittel zum Verbinden mit einem distalen Ende eines Infusionsschlauch.

9. Infusionskatheter (500) mit:
- einem distalen Ende (502) zum Einführen in einen Patienten;
- einem proximalen Ende (504) zum Verbinden mit einem distalen Ende eines Infusionsschlauchs,
**dadurch gekennzeichnet, dass** er zwischen dem distalen Ende (502) und dem proximalen Ende (504) eine Vorrichtung (506-509) zur Entnahme von Körperflüssigkeit nach einem der Ansprüche 1 bis 8 umfasst und so ausgelegt ist, dass, in Verwendung, die Intubationszone in einem vertikalen maximale bestimmten Abstand (Dvₘₐₓ) von dem proximalen Ende des Katheters angeordnet ist.

10. Infusionsschlauch (606) mit:
- einem proximalen Ende (602) zum Verbinden mit einem Behälter einer ersten Infusionslösung;
- einem distalen Ende (604) zum Verbinden mit einem proximalen Ende eines Infusionskatheters, der in einen Patienten eingeführt ist;
**dadurch gekennzeichnet, dass** er zwischen dem proximalen Ende (602) und dem distalen Ende (604) eine Vorrichtung (606-609) zur Entnahme von Körperflüssigkeit nach einem der Ansprüche 1 bis 8 umfasst und so ausgelegt ist, dass, in Verwendung, die Intubationszone in einem vertikalen maximalen bestimmten Abstand (Dvₘₐₓ) von dem proximalen Ende des Katheters angeordnet ist.

11. Satz zur Entnahme von Körperflüssigkeit, **dadurch gekennzeichnet, dass** er umfasst:
- einen Entnahmekanal (300) mit einem proximalen Ende (302) zum Verbinden mit einem Entnahmebehälter (303) und einem distalen Ende (301), und
- einer Entnahmevorrichtung nach einem der Ansprüche 1 bis 8.

12. Satz zur Entnahme von Körperflüssigkeit nach Anspruch 11, der ein Verhältnis zwischen dem Innendurchmesser (Dc) des Entnahmekanals und dem Außendurchmesser (Dt) der röhrenartigen Struktur der Entnahmevorrichtung von kleiner 1, typischerweise zwischen 1/20 und 1/3, besitzt.

13. Satz zur Entnahme von Körperflüssigkeit nach einem der Ansprüche 11 oder 12, in dem der Entnahmekanal (300) eine Länge zwischen seinen zwei Enden zwischen 10 cm und 100 cm, vorzugsweise zwischen 20 cm und 50 cm, besitzt.

14. Satz zur Entnahme von Körperflüssigkeit nach einem der Ansprüche 11 bis 13, der ferner ein Mittel (700) zur Analyse der entnommenen Körperflüssigkeit umfasst.

15. Satz zur Entnahme von Körperflüssigkeit nach Anspruch 14, in dem das Analysemittel (700) ferner dazu geeignet ist, eine bestimmte Lösungsprobe zu analysieren und die entnommene Körperflüssigkeit mit der bestimmten Lösungsprobe zu vergleichen.

16. Satz zur Entnahme von Körperflüssigkeit nach einem der Ansprüche 14 oder 15, in dem das Analysemittel (700) eine Reaktionskammer (710) und ein Erfassungsmittel (720, 721) umfasst.

17. Satz zur Entnahme von Körperflüssigkeit nach einem der Ansprüche 14 bis 16, der ferner ein Mittel (730, 731) zur Fließsteuerung der Lösung einer zweiten Infusion umfasst, wobei das Analysemittel (700, 731) dazu geeignet ist, an das Mittel (730, 731) zur Fließsteuerung der Lösung eine Information des Vergleichs (I_{C}) der entnommenen Körperflüssigkeit (304) mit der bestimmten Lösungsprobe (810) zu übertragen.

18. Satz zur Entnahme von Körperflüssigkeit nach Anspruch 17, der ein Mittel (740, 741) zur Anzeige der Information des Vergleichs (I_{C}) zwischen der entnommenen Körperflüssigkeit (304) und der bestimmten Lösungsprobe (810) umfasst.

19. Satz zur Entnahme von Körperflüssigkeit nach einem der Ansprüche 17 oder 18, in dem das Analysemittel (700, 720) dazu geeignet ist, das Mittel (730, 731) zur Fließsteuerung der Lösung so zu steuern, dass es das Fließen der Lösung der zweiten Infusion blockiert, wenn die Körperflüssigkeit und das Produkt der zweiten Infusion nicht kompatibel sind, und so, dass es das Fließen der Lösung der zweiten Infusion freigibt wenn die Körperflüssigkeit und das Produkt der zweiten Infusion kompatibel sind.

20. Satz zur Entnahme von Körperflüssigkeit nach einem der Ansprüche 17 oder 18, in dem das Analysemittel (700, 720) dazu geeignet ist, das Mittel (730, 731) zur Fließsteuerung der Lösung so zu steuern, dass es das Fließen der Lösung der zweiten Infusion automatisch erzeugt, wenn die Körperflüssigkeit und das Produkt der zweiten Infusion kompatibel sind, und dass es das Fließen der zweiten Infusion nicht steuert, wenn sie nicht kompatibel sind.

21. Satz zur Entnahme von Körperflüssigkeit nach einem der Ansprüche 11 bis 20, ferner umfassend einen Entnahmebehälter (303).

22. Satz zur Entnahme von Körperflüssigkeit nach einem der vorhergehenden Ansprüche, in dem der Entnahmebehälter ein Blutantikoagulans enthält.

23. Satz zur Entnahme von Körperflüssigkeit nach einem der Ansprüche 21 oder 22, in dem der Entnahmebehälter ein Behälter (2000-3000) mit zwei Kammern (2010-2020, 3010-3020) ist, die durch ein Nichtrückkehrsystem (2030, 3050) getrennt sind.

24. Satz zur Entnahme von Körperflüssigkeit nach einem der Ansprüche 21 bis 23, in dem der Entnahmebehälter ein verformbarer und mechanisch betätigbarer Behälter sein kann.

25. Satz zur Entnahme von Körperflüssigkeit nach einem der Ansprüche 21 bis 24, in dem der Entnahmebehälter zuvor vakuumdicht unter Druck gesetzt und mit einer Klammer in diesem Zustand gehalten wird.

26. Satz zur Entnahme von Körperflüssigkeit nach einem der Ansprüche 11 bis 25, der einen Entnahmekatheter (500) nach Anspruch 9 umfasst, der mit der Entnahmevorrichtung ausgestattet ist.

27. Satz zur Entnahme von Körperflüssigkeit nach einem der Ansprüche 11 bis 25, der eine Infusionsschlauch (600) nach Anspruch 10 umfasst, der mit der Entnahmevorrichtung ausgestattet ist.

28. Verfahren zur Verwendung eines Satzes zur Entnahme von Körperflüssigkeit nach einem der Ansprüche 11 bis 27, **dadurch gekennzeichnet, dass** es die folgenden Schritte umfasst:
- Integrieren der Entnahmevorrichtung (200, 400, 500, 600) in einen zuvor an einem Patienten, der mit einem Infusionskatheter (104) versorgt ist, installierten Fluidkreislauf einer Infusion (100) in einem bezüglich des Körpers (B) des Menschen äußeren Teil eines Fluidkreislaufs;
- Halten des Teils des Entnahmekanals (300), der das distale Ende (301) umfasst, im Inneren der röhrenartigen Struktur, und zwar derart, dass das distale Ende (301) zu dem Infusionskatheter (104), in Strömungsrichtung (F1) der Infusionslösung (102) von einem Infusionslösungsbehälter (101) zu dem Infusionskatheter (104), gerichtet wird;
- Positionieren des Entnahmebehälters (303) auf einer Höhe (H_{c}), die niedriger als diejenige (Hₖₐₜ) des Katheters des Patienten ist, und zwar derart, dass die Körperflüssigkeit in dem Entnahmekanal (300) in Gegenrichtung (F2) zu der Infusionslösung und dann in Richtung des Entnahmebehälters fließt; und
- Halten des Entnahmebehälters in einer Höhe unterhalb derjenigen des Katheters des Patienten während einer Dauer, die ausreichend ist, um in dem Entnahmebehälter ein Volumen zu gewinnen, das ausreichend ist, um eine Entnahme der Körperflüssigkeit zu erreichen.

29. Verfahren nach Anspruch 28, zur Verwendung eines Satzes zur Entnahme von Körperflüssigkeit nach einem der Ansprüche 9 bis 20, umfassend eine Entnahmevorrichtung nach einem der Ansprüche 3 bis 8, umfassend einen Einführungsschritt, über das Einführungsmittel (204, 104), des distalen Endes (301) des Entnahmekanals (300) in die röhrenartige Struktur (202, 402) der Entnahmevorrichtung, und zwar derart, dass das distale Ende in Strömungsrichtung (F1) der Infusionslösung zu dem Infusionskatheter (104) gerichtet ist.

30. Verfahren nach einem der Ansprüche 28 oder 29, in dem die Entnahmevorrichtung (200, 400, 500, 600) so ausgelegt ist, dass, in Verwendung, die Intubationszone in einem vertikalen maximalen vorbestimmten Abstand (Dvₘₐₓ) von dem proximalen Ende des Katheters angeordnet ist.

31. Verfahren nach Anspruch 30, in dem der vertikale maximale vorbestimmte Abstand (Dvₘₐₓ) zwischen 0 cm und 50 cm beträgt.

32. Verfahren nach einem der Ansprüche 28 bis 31, in dem das distale Ende des Entnahmekanals in einem vorbestimmten Abstand von dem proximalen Ende des Infusionskatheters angeordnet ist, wobei der genannte Abstand als "Einmündungsabstand" bezeichnet ist und zwischen 0 cm und 20 cm, vorzugsweise zwischen 0 cm und 3 cm. beträgt.

33. Verfahren nach einem der Ansprüche 28 bis 32, ferner umfassend einen Schritt der Fluidverbindungsherstellung des Entnahmebehälters mit einem Mittel zur Analyse der entnommenen Körperflüssigkeit und einen Schritt zur Analyse der entnommenen Körperflüssigkeit.

34. Verfahren nach Anspruch 33, ferner umfassend einen Schritt zur Analyse und zum Vergleich einer bestimmten Lösungsprobe mit der entnommenen Körperflüssigkeit.

35. Verfahren nach Anspruch 34, ferner umfassend einen Schritt zur Erzeugung einer Information des Vergleichs zwischen der entnommenen Körperflüssigkeit und der bestimmten Lösungsprobe.

36. Verfahren nach Anspruch 35, ferner umfassend einen Schritt zur Herstellung einer Fluidverbindung von einer zweiten bestimmten Infusionslösung mit dem Fluidkreislauf der zuvor hergestellten Infusion.

37. Verfahren nach Anspruch 36, ferner umfassend einen Schritt zur Übertragung, mit Hilfe eines Mittels zur Steuerung des Produktflusses der zweiten Infusion, der Information des Vergleichs der entnommenen Körperflüssigkeit mit der bestimmten Lösungsprobe.

38. Verfahren nach Anspruch 37, ferner umfassend einen Schritt der Anzeige einer Information des Vergleichs der entnommenen Körperflüssigkeit mit der bestimmten Lösungsprobe.

39. Verfahren nach einem der Ansprüche 37 oder 38, ferner umfassend einen Schritt der Freigabe des Flusses der Lösung der zweiten Infusion, wenn die Information des Vergleichs anzeigt, dass die Körperflüssigkeit und das Produkt der zweiten Infusion kompatibel sind, und der Blockierung des Flusses der Lösung der zweiten Infusion, wenn die Information des Vergleichs anzeigt, dass die Körperflüssigkeit und das Produkt der zweiten Infusion kompatibel nicht sind.

40. Verfahren nach einem der Ansprüche 37 oder 38, ferner umfassend einen Schritt zur automatischen Erzeugung des Flusses der Produkts der zweiten Infusion, wenn die Information des Vergleichs anzeigt, dass die Körperflüssigkeit und das Produkt der zweiten Information kompatibel sind, und der Blockierung Flusses der Produkts der zweiten Infusion, wenn die Information des Vergleichs anzeigt, dass die Körperflüssigkeit und das Produkt der zweiten Information kompatibel nicht sind.

## Claims

1. A sample collection device (200, 400, 506, 509, 606, 609, 1000) for collecting a sample of a body fluid, such as blood, intended to be incorporated into a fluid circuit of a perfusion (100) of a patient equipped with a perfusion catheter (104, 504), and comprising a tubular structure (202, 402, 501, 507, 601, 607, 1002) for connection to the fluid circuit of the perfusion equipped:
- with a zone (Zi) of intubation, in use, of a sample collection channel (300, 1100) comprising a distal end (301); and
- with a means (203, 503, 603, 610, 1004, 1004a) for holding, in use, in the tubular structure, the portion of the sample collection channel (300, 1100) comprising a distal end (301, 1101), so that said distal end (301, 1101) is pointed toward the perfusion catheter (104), in the flow direction (F1) of the perfusion product (102), from a container (101) of perfusion product to the perfusion catheter (104).

2. The sample collection device (1000) as claimed in claim 1, wherein the portion comprising the distal end (1101) of the sample collection channel (1100) is fixed in the tubular structure (1002), and a portion (1003) comprising a proximal end of the sample collection channel (1100) emerges outside of the tubular structure (1002) level with the intubation zone (Zi).

3. The sample collection device (200, 400, 506, 509, 606, 609) as claimed in claim 1, wherein the intubation zone comprises an insertion means (204, 404) suitable for enabling, in use, the insertion, into the tubular structure, of the portion of the sample collection channel (300) comprising the distal end (301).

4. The sample collection device as claimed in any one of claims 1 to 3, wherein the intubation zone is arranged so that, in use, it is positioned at a given maximum vertical distance (Dvₘₐₓ) from the proximal end of the catheter.

5. The sample collection device as claimed in claim 4, wherein the given maximum vertical distance (Dvₘₐₓ) is between 0 cm and 50 cm.

6. The sample collection device as claimed in any one of claims 3 to 5, wherein the insertion means is chosen from:
- a membrane (404) made of a leaktight material that retains its leaktightness after having been pierced; and
- a leaktight connector.

7. The sample collection device as claimed in claim 6, wherein the leaktight material that retains its leaktightness after having been pierced is chosen from a silicone polymer, such as polydimethylsiloxane (PDMS), polymethyl methacrylate (PMMA), polyvinyl chloride (PVC) and Tygon®.

8. The sample collection device as claimed in any one of claims 1 to 7, comprising, in addition:
- a means of connection to a proximal end of a perfusion catheter, and
- a means of connection to a distal end of a perfusion tubing.

9. A perfusion catheter (500) comprising:
- a distal end (502) intended to be inserted into a patient;
- a proximal end (504) intended to be connected with a distal end of a perfusion tubing,
**characterized in that** it comprises, between the distal end (502) and the proximal end (504), a device (506-509) for collecting a sample of body fluid as claimed in any one of claims 1 to 8, arranged so that, in use, the intubation zone is positioned at a given maximum vertical distance (Dvₘₐₓ) from the proximal end of the catheter.

10. A perfusion tubing (600) comprising:
- a proximal end (602) intended to be connected to a container of a first perfusion product;
- a distal end (604) intended to be connected with a proximal end of a perfusion catheter inserted into a patient;
**characterized in that** it comprises, between the proximal end (602) and the distal end (604), a device (606-609) for collecting a sample of body fluid as claimed in any one of claims 1 to 8, arranged so that, in use, the intubation zone is positioned at a given maximum vertical distance (Dvₘₐₓ) from the proximal end of the catheter.

11. A kit for collecting a sample of a body fluid, **characterized in that** it comprises:
- a sample collection channel (300) comprising a proximal end (302) intended to be connected to a sample collection container (303), and a distal end (301); and
- a sample collection device as claimed in any one of claims 1 to 8.

12. The kit for collecting a sample of a body fluid as claimed in claim 11, having a ratio between the internal diameter (Dc) of the sample collection channel and the external diameter (Dt) of the tubular structure of the sample collection device of less than 1, typically between 1/20 and 1/3.

13. The kit for collecting a sample of a body fluid as claimed in either one of claims 11 and 12, wherein the sample collection channel (300) has a length between its two ends of between 10 cm and 100 cm, preferably between 20 cm and 50 cm.

14. The kit for collecting a sample of a body fluid as claimed in any one of claims 11 to 13, comprising, in addition, a means (700) for analyzing the collected body fluid.

15. The kit for collecting a sample of a body fluid as claimed in claim 14, wherein the analysis means (700) is also capable of analyzing a sample of given product and of comparing the collected body fluid and the sample of given product.

16. The kit for collecting a sample of a body fluid as claimed in either of claims 14 and 15, wherein the analysis means (700) comprises a reaction chamber (710) and a detection means (720, 721).

17. The kit for collecting a sample of a body fluid as claimed in any one of claims 14 to 16, comprising, in addition, a means (730, 731) for controlling the flow of the product of a second perfusion, the analysis means (700, 720) being capable of transmitting to the flow control means (730, 731) comparison information (I_{C}) of the collected body fluid (304) and the sample of given product (810).

18. The kit for collecting a sample of a body fluid as claimed in claim 17, comprising a means (740, 741) for displaying the comparison information (I_{C}) of the collected body fluid (304) and the sample of given product (810).

19. The kit for collecting a sample of a body fluid as claimed in either one of claims 17 and 18, wherein the analysis means (700, 720) is capable of controlling the flow control means (730, 731) so that it blocks the flow of the product of the second perfusion if the body fluid and the product of the second perfusion are incompatible, and so that it permits the flow of the product of the second perfusion if the body fluid and the product of the second perfusion are compatible.

20. The kit for collecting a sample of a body fluid as claimed in either one of claims 17 and 18, wherein the analysis means (700, 720) is capable of controlling the flow control means (730, 731) so that it automatically generates the flow of the product of the second perfusion if the body fluid and the product of the second perfusion are compatible, and so that it does not generate the flow of the product of the second perfusion if the body fluid and the product of the second perfusion are incompatible.

21. The kit for collecting a sample of a body fluid as claimed in any one of claims 11 to 20, comprising, in addition, a sample collection container (303).

22. The kit for collecting a sample of a body fluid as claimed in the preceding claim, wherein the sample collection container comprises a blood anticoagulant means.

23. The kit for collecting a sample of a body fluid as claimed in either one of claims 21 and 22, wherein the sample collection container is a container (2000-3000) having two compartments (2010-2020, 3010-3020) separated by a non-return system (2030, 3030).

24. The kit for collecting a sample of a body fluid as claimed in any one of claims 21 to 23, wherein the sample collection container may be container that can be deformed and actuated mechanically.

25. The kit for collecting a sample of a body fluid as claimed in any one of claims 21 to 24, wherein the sample collection container is put under vacuum beforehand and kept in this state by a clamp.

26. The kit for collecting a sample of a body fluid as claimed in any one of claims 11 to 25, comprising a perfusion catheter (500) as claimed in claim 9, equipped with the sample collection device.

27. The kit for collecting a sample of a body fluid as claimed in any one of claims 11 to 25, comprising a perfusion tubing (600) as claimed in claim 10, equipped with the sample collection device.

28. A process for implementing a kit for collecting a sample of a body fluid as claimed in any one of claims 11 to 27, **characterized in that** it comprises the following steps:
- incorporating the sample collection device (200, 400, 500, 600) into a fluid circuit of a perfusion (100) previously fitted to a patient equipped with a perfusion catheter (104), in an external portion of the fluid circuit relative to the body of the patient (B);
- holding, inside the tubular structure, the portion of the sample collection channel (300) comprising the distal end (301), so that said distal end (301) is pointed toward the perfusion catheter (104), in the flow direction (F1) of the perfusion product (102), from a container (101) of perfusion product to the perfusion catheter (104);
- bringing the sample collection container (303) to a height (H_{c}) below that (H_{cat}) of the catheter of the patient, so that the body fluid flows into the sample collection channel (300), countercurrently (F2) compared to the perfusion product, then toward the sample collection container; and
- holding the sample collection container at a height below that of the catheter of the patient for a sufficient time to obtain, in the sample collection container, a volume sufficient to constitute a collection of a sample of body fluid.

29. The process as claimed in claim 28 for implementing a kit for collecting a sample of a body fluid as claimed in any one of claims 9 to 20, comprising a sample collection device as claimed in any one of claims 3 to 8, comprising a step of inserting, via the insertion means (204, 404), the distal end (301) of the sample collection channel (300) into the tubular structure (202, 402) of the sample collection device, so that said distal end (301) is pointed toward the perfusion catheter (104), in the flow direction (F1) of the perfusion product.

30. The process as claimed in either of claims 28 and 29, wherein the sample collection device (200, 400, 500, 600) is arranged so that, in use, the intubation zone is positioned at a given maximum vertical distance (Dvₘₐₓ) from the proximal end of the catheter.

31. The process as claimed in claim 30, wherein the given maximum vertical distance (Dvₘₐₓ) is between 0 cm and 50 cm.

32. The process as claimed in one of claims 28 to 31, wherein the distal end of the sample collection channel is arranged at a given distance from a proximal end of the perfusion catheter, said distance being referred to as the "butt-joining distance" and being between 0 cm and 20 cm, preferably between 0 cm and 3 cm.

33. The process as claimed in any one of claims 28 to 32, comprising, in addition, a step of placing the sample collection container in fluid communication with a means of analyzing the collected body fluid, and a step of analyzing the collected body fluid.

34. The process as claimed in claim 33, comprising, in addition, a step of analyzing and comparing a sample of given product and the collected body fluid.

35. The process as claimed in claim 34, comprising, in addition, a step of generating comparison information of the collected body fluid and the sample of given product.

36. The process as claimed in claim 35, comprising, in addition, a step of placing a second perfusion of given product in fluid communication with the fluid circuit of the previously installed perfusion.

37. The process as claimed in claim 36, comprising, in addition, a step of transmitting, to a means for controlling the flow of the product of the second perfusion, the comparison information of the collected body fluid and the sample of given product.

38. The process as claimed in claim 37, comprising, in addition, a step of displaying comparison information of the collected body fluid and the sample of given product.

39. The process as claimed in either one of claims 37 and 38, comprising, in addition, a step of permitting the flow of the product of the second perfusion if the comparison information indicates that the body fluid and the product of the second perfusion are compatible, and of blocking the flow of the product of the second perfusion if the comparison information indicates that the body fluid and the product of the second perfusion are incompatible.

40. The process as claimed in either one of claims 37 and 38, comprising, in addition, a step of automatically generating the flow of the product of the second perfusion if the comparison information indicates that the body fluid and the product of the second perfusion are compatible, and of blocking the flow of the product of the second perfusion if the comparison information indicates that the body fluid and the product of the second perfusion are incompatible.
